# EUROPEAN PATENT APPLICATION

(11) **EP 4 721 688 A1**
(43) Date of publication of application: **08.04.2026**
(21) Application number: 24811228.6
(22) Date of filing: 29.02.2024
(51) Int. Cl.: A61B 34/10, A61C 1/08, A61C 8/00, G16H 30/20, G16H 30/40

(54) **METHOD AND DEVICE FOR DESIGNING IMPLANT SURGICAL GUIDE FOR BONE REDUCTION**

(30) Priority: 24.05.2023 KR 20230067163; 25.05.2023 KR 20230067566
(71) Applicant: Osstem Implant Co., Ltd., Gangseo-gu Seoul 07789 (KR)
(72) Inventor: KIM, Jong Moon, Gunpo-si Gyeonggi-do 15827 (KR)
(74) Representative: Klunker IP Patentanwälte PartG mbB
(86) International application number: PCT/KR2024/002639
(87) International publication number: WO 2024/242289

(57) **Abstract**

Disclosed are a method and a device for designing an implant surgical guide for bone reduction. The method and the device for designing an implant surgical guide for bone reduction, according to an embodiment, can quickly and accurately perform bone reduction by designing a virtual bone reduction guide shape including a guide rail, when bone reduction is required for dental implant surgery.

## Description

### [Technical Field]

The present invention relates to dental image data processing technology, and more particularly, to implant surgical guide design and user interface technology for bone reduction.

### [Background Art]

In general, dentures or prosthetics are artificial periodontal tissues placed in the oral cavity to replace missing natural teeth and artificially restore their appearance and function. In the case of an edentulous patient or a patient with poor remaining teeth, dental restoration is performed using dental restorations such as complete dentures or complete prostheses. However, after a long period of time has elapsed since the patient's tooth was extracted, the bone width in the anterior region may become narrow due to the direction of bone resorption. In such a case, when performing implant surgery, bone around the implant can be secured by reducing the narrow bone in the anterior region.

As another example, bone reduction may be required when there is insufficient space between the patient's upper jaw and lower jaw for placing a dental restoration, or when it is desired to prevent exposure of the boundary between the dental restoration and the gingiva when smiling.

As yet another example, vertical bone grafting in the posterior region due to loss of posterior teeth is highly likely to result in bone resorption. Therefore, in this case as well, bone reduction of the anterior region is necessary.

### [Disclosure]

### [Technical problem]

According to an embodiment, when bone reduction is required for dental implant surgery, a method for designing and implant surgical guide for bone reduction and a device therefor are proposed.

### [Technical Solution]

A method for designing an implant surgical guide, according to an embodiment, includes the steps of: placing a virtual implant into dental image data; generating a virtual implant surgical guide shape including a virtual implant guide hole; generating a virtual bone reduction guide shape for guiding bone reduction parallel to an occlusal plane; generating a virtual guide rail having an insertion portion and protruding along an outer periphery of the virtual bone reduction guide shape; and designing a coupled form of the virtual implant surgical guide shape and the virtual bone reduction guide shape.

The step of placing the virtual implant may include displaying a placement position of the virtual implant so as to match a line or plane of a bone reduction interface whose position is adjusted in accordance with a user operation signal through the bone reduction interface.

The bone reduction interface may be in the form of a line or plane parallel to the occlusal plane.

The step of generating the virtual implant surgical guide shape may include forming the virtual implant guide hole at a position spaced apart by a predetermined distance from the top end portion of the placed virtual implant.

The step of generating the virtual implant surgical guide shape comprises forming the virtual implant guide hole such that an axis of the placed virtual implant coincides with a center point of the virtual implant guide hole.

The step of generating the virtual bone reduction guide shape may include generating an outermost contour line of the virtual bone reduction guide shape on the basis of a position of the virtual implant guide hole.

The step of generating the virtual bone reduction guide shape may include generating an outermost contour line of the virtual bone reduction guide shape on the basis of a line or plane of the bone reduction interface whose position is adjusted in accordance with a user operation signal.

The virtual guide rail may have a plane parallel to the occlusal plane and a vertical surface perpendicular to the occlusal plane.

The virtual guide rail may be generated within a movable range of a bone reduction interface and the movable range of the bone reduction interface is within a range that does not invade at least one of anatomical structures among a nerve canal, maxillary sinus, maxilla, and mandible.

The virtual guide rail may be formed at a position spaced apart by a predetermined distance vertically and horizontally from a bone reduction interface line or plane.

The step of designing the coupled form may include designing the coupled form of the virtual implant surgical guide shape and the virtual bone reduction guide shape such that, when an actual implant surgical guide is fastened to an actual bone reduction guide, the actual implant surgical guide is coupled in a manner where the actual implant surgical guide is fitted into the actual bone reduction guide by physical force.

The step of designing the coupled form may include designing the coupled form of the virtual implant surgical guide shape and the virtual bone reduction guide shape such that an actual bone reduction guide is mounted into a cylindrical groove of an actual implant surgical guide.

The step of designing the coupled form may include designing the coupled form of the virtual implant surgical guide shape and the virtual bone reduction guide shape such that an actual bone reduction guide and an actual implant surgical guide are coupled in an omega shape.

The method for designing an implant surgical guide may further include the step of generating a virtual anchor pin drilling hole in the virtual bone reduction guide shape.

The method for designing an implant surgical guide may further include the steps of: inserting a guiding mechanism of a bone reduction device into an actual insertion portion of an actual guide rail manufactured by outputting the virtual guide rail; moving the guiding mechanism of the bone reduction device, inserted into the insertion portion, along a movement path constrained by the actual guide rail; and reducing bone protruding from an opening of the actual bone reduction guide, with a cutting mechanism coupled to the guiding mechanism moving together along the movement path.

The method for designing an implant surgical guide may further include the steps of: obtaining length information of a guide drill for implant surgery; determining whether to integrate the virtual bone reduction guide shape and the virtual implant surgical guide according to the obtained length of the guide drill; in response to determining to integrate the virtual bone reduction guide shape and the virtual implant surgical guide shape, generating the virtual bone reduction guide shape and the virtual implant surgical guide shape in an integrated manner from the dental image data; and displaying the generated data.

The determining of whether to integrate may include the steps of: determining a bone height before bone reduction with respect to a position of the virtual implant placed in the dental image data; checking the length of the guide drill; and comparing the bone height and the length of the guide drill; and, according to a comparing result, recommending whether to integrate or to separate the virtual bone reduction guide shape and the virtual implant surgical guide.

The step of generating in an integrated manner may include the steps of: generating the virtual implant surgical guide shape including the virtual implant guide hole; generating the virtual bone reduction guide shape; designing a coupled form of the virtual bone reduction guide shape and the virtual implant surgical guide shape; generating a bone reduction guide line corresponding to a bone reduction line in the virtual bone reduction guide shape and the virtual implant surgical guide shape; generating an implant surgical guide line parallel to and at a predetermined distance away from the bone reduction guide line; separating the virtual bone reduction guide shape and the virtual implant surgical guide shape by removing a region between the bone reduction guide line and the implant surgical guide line; and integrating the virtual bone reduction guide shape and the virtual implant surgical guide shape by combining the bone reduction guide line and the implant surgical guide line through a coupling portion.

A device for designing an implant surgical guide, according to another embodiment, includes a data acquisition unit configured to obtain dental image data for implant surgery; a control unit configured to generate a virtual implant surgical guide shape including a virtual implant guide hole, to generate a virtual bone reduction guide shape for guiding bone reduction parallel to the occlusal plane, to generate a virtual guide rail having an insertion portion and protruding along an outer periphery of the virtual bone reduction guide shape, and to design a coupled form of the virtual implant surgical guide shape and the virtual bone reduction guide shape; and a display unit configured to display data including the virtual implant surgical guide shape and the virtual bone reduction guide shape.

### [Effects of the Invention]

According to a method for designing an implant surgical guide and a device therefor according to an embodiment, a virtual bone reduction guide shape can be generated. By using an actual bone reduction guide manufactured by outputting the virtual bone reduction guide shape, actual bone can be reduced.

In addition, the method and device for designing an implant surgical guide according to an embodiment can design a virtual bone reduction guide shape including a virtual guide rail. Accordingly, bone reduction can be performed quickly and accurately by using an actual guide rail manufactured by outputting the virtual guide rail.

Moreover, the method and device for designing an implant surgical guide according to an embodiment can generate a virtual bone reduction guide shape configured to allow bone reduction to be performed parallel to the occlusal plane. For example, when the plane of the virtual guide rail is parallel to the occlusal plane and the vertical surface thereof is perpendicular to the occlusal plane, bone can be reduced parallel to the occlusal plane using a bone reduction device. Furthermore, virtual implant placement can be formed perpendicular to the occlusal plane. Accordingly, when manufacturing a prosthesis thereafter, occlusal force is directly transferred to the implant, thereby enabling the production of a stable prosthesis.

In addition, the method and device for designing an implant surgical guide according to an embodiment presents a coupling structure of a virtual implant placement guide shape and a virtual bone reduction guide shape, thereby allowing the virtual implant placement guide shape and the virtual bone reduction guide shape to be stably coupled.

Meanwhile, when the virtual implant surgical guide shape and the virtual bone reduction guide shape are each designed separately and then manufactured, it takes a significant amount of time to manufacture components related to dental restoration. As a result, the overall period for dental restoration increases. In contrast, the method and device for designing an implant surgical guide according to an embodiment presents a method for designing and manufacturing a virtual implant surgical guide shape and a virtual bone reduction guide shape as an integrated structure, thereby resolving the above-described problems that occur when designing and manufacturing them separately.

Moreover, according to the method and device for designing an implant surgical guide according to an embodiment, when bone reduction is required for dental implant surgery, it is possible to determine whether to integrate the virtual implant surgical guide shape and the virtual bone reduction guide shape according to the length of a guide drill and to inform a user of the result. Accordingly, user convenience can be increased.

If the length of the guide drill is not taken into account, the integrated virtual implant surgical guide shape and virtual bone reduction guide shape cannot be used. Therefore, the virtual implant surgical guide shape and the virtual bone reduction guide shape must be separately redesigned and produced again. However, the method and device for designing an implant surgical guide according to an embodiment determines whether to integrate the virtual implant surgical guide shape and the virtual bone reduction guide shape by taking into account the length of the guide drill when the length of the guide drill is not otherwise considered. Accordingly, the aforementioned problem that occurs when the length of the guide drill is not taken into consideration can be resolved.

Furthermore, in the course of designing the virtual implant surgical guide shape and the virtual bone reduction guide shape, the method and device for designing an implant surgical guide according to an embodiment determines and provides an implant placement guide line parallel to the bone reduction line, thereby resolving the problem of reduced registration accuracy that occurs when manually setting the implant placement guide line.

### [Description of Drawings]

FIG. 1 is a diagram illustrating a configuration of a device for designing an implant surgical guide according to an embodiment of the present invention.
FIG. 2 is a view illustrating dental image data according to an embodiment of the present invention.
FIG. 3 is a view illustrating a screen for separating anatomical structures from dental image data according to an embodiment of the present invention.
FIG. 4 is a view illustrating a screen for registering first CT data and scan data of a patient according to an embodiment of the present invention.
FIGS. 5 and 6 are views illustrating a screen for setting an occlusal plane using the first CT data of first registered data according to an embodiment of the present invention.
FIGS. 7 to 10 are views illustrating arch line generation screens according to an embodiment of the present invention.
FIGS. 11 to 15 are views illustrating a virtual crown arrangement process according to an embodiment of the present invention.
FIG. 16 is a view illustrating a movable range of a bone reduction interface according to an embodiment of the present invention.
FIG. 17 is a view illustrating a screen displaying, through a user interface, a permissible position for virtual implant placement, according to an embodiment of the present invention.
FIG. 18 and FIG. 19 are views illustrating a screen for placing a virtual implant according to an embodiment of the present invention.
FIG. 20 is a view illustrating a screen displaying, in a distinguishable manner, a permissible position for virtual implant placement and a non-permissible position for virtual implant placement according to an embodiment of the present invention.
FIG. 21 is a view illustrating a bone width that serves as a criterion for distinguishing between a permissible position for virtual implant placement and a non-permissible position for virtual implant placement according to an embodiment of the present invention.
FIG. 22 is a view illustrating a screen for placing a virtual implant inclined at a predetermined angle with respect to a tooth axis, according to an embodiment of the present invention.
FIG. 23 is a view illustrating a screen displaying a permissible placement position of a virtual implant having a corresponding diameter, according to a change in the diameter of a virtual implant by a user, according to an embodiment of the present invention.
FIG. 24 is a view illustrating a screen displaying a change in a virtual implant placement position when a user changes the diameter of a virtual implant to be placed, according to an embodiment of the present invention.
FIG. 25 is a view illustrating a screen displaying all virtual implant placement positions selected by the user according to an embodiment of the present invention.
FIG. 26 is a view illustrating an example of generating a virtual implant surgical guide shape and a virtual bone reduction guide shape according to an embodiment of the present invention.
FIG. 27 is a view illustrating a bone reduction device according to an embodiment of the present invention.
FIG. 28 is a view illustrating a guide rail form of the virtual bone reduction guide shape according to an embodiment of the present invention.
FIGS. 29 to 31 are views illustrating examples of designing a coupled form of a virtual implant placement guide shape and a virtual bone reduction guide shape according to various embodiments of the present invention.
FIG. 32 is a view illustrating an example of generating an anchor pin drilling hole in the virtual bone reduction guide shape according to an embodiment of the present invention.
FIG. 33 and FIG. 34 are views illustrating screens that recommend integrated generation or separate generation of a virtual bone reduction guide shape and a virtual implant surgical guide shape according to the length of a guide drill, according to an embodiment of the present invention.
FIG. 35 is a view illustrating a guided surgery kit selection screen according to an embodiment of the present invention.
FIG. 36 is a view illustrating a screen for integrating a virtual bone reduction guide shape and a virtual implant surgical guide shape according to an embodiment of the present invention.
FIG. 37 is a flowchart illustrating a method for designing an implant surgical guide according to an embodiment of the present invention.
FIG. 38 is a view illustrating a process of generating a virtual implant surgical guide shape and a virtual bone reduction guide shape according to an embodiment of the present invention.

### [Mode of the Invention]

The advantages and features of the present invention and the manner of achieving the advantages and features will become apparent with reference to embodiments described in detail below together with the accompanying drawings. However, the present invention may be implemented in many different forms and should not be construed as being limited to the embodiments set forth herein, and the embodiments are provided such that this disclosure will be thorough and complete and will fully convey the scope of the present invention to those skilled in the art, and the present invention is defined only by the scope of the appended claims. Throughout the entire specification, the same or like reference numerals designate the same or like elements.

In describing the example embodiments, a detailed description of related known configurations or functions incorporated herein will be omitted when it is determined that the detailed description thereof may unnecessarily obscure the subject matter of the present invention. The terms which will be described below are terms defined in consideration of the functions in the present invention, and may be different according to users, intentions of the users, or customs. Therefore, the definitions of the terms used herein should follow contexts disclosed herein.

Hereinafter, embodiments of the present invention will be described in detail with reference to the accompanying drawings. However, the example embodiments of the present invention may be modified in various different forms, and the scope of the present invention is not limited to the example embodiments described below. Embodiments of the present invention are provided to aid those skilled in the art in the explanation and the understanding of the present invention.

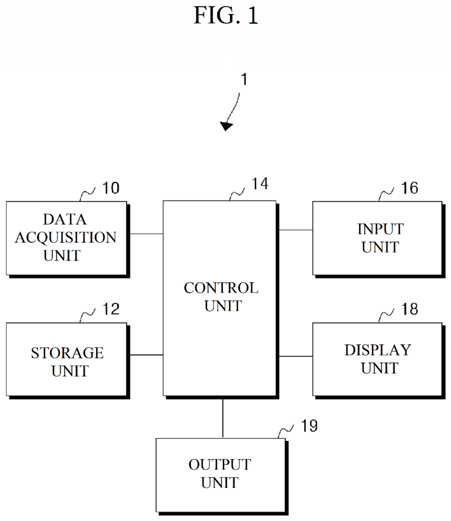

FIG. 1 is a diagram illustrating a configuration of a device for designing an implant surgical guide according to an embodiment of the present invention.

Referring to FIG. 1, a device 1 for designing an implant surgical guide is an electronic device capable of executing a medical image processing program. The electronic device may include a personal computer (PC), a notebook computer, a tablet computer, a smartphone, and the like. Examples of the medical image processing program include a guide design program, a scanning program, a CAD program, and the like. In addition, the program may be applied not only to dental implant surgery but also to general medical image processing applications. Hereinafter, for convenience of description, a guide design program for dental implant surgery will be described as an example. However, it should be understood that the invention is equally applicable to other programs as long as image processing is possible.

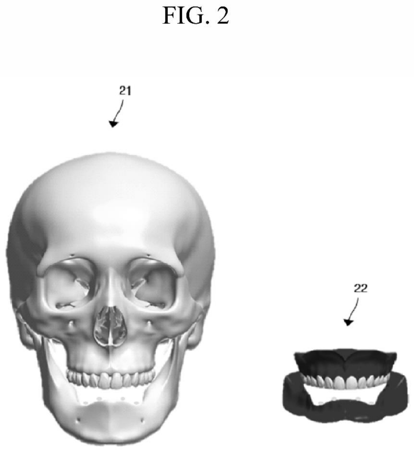

The image processing procedure using the medical image processing program includes: registering a surgery patient; acquiring dental image data of the registered patient; generating an arch line from the dental image data and creating a panoramic image using the arch line; setting the position of a crown in the dental image data; setting a placement position of a virtual implant; generating a virtual implant surgical guide shape; and outputting an actual implant surgical guide. The dental image data may include CT data, scan data, and the like. An example of dental image data will be described below with reference to FIG. 2.

The device 1 for designing an implant surgical guide according to an embodiment includes a data acquisition unit 10, a storage unit 12, a control unit 14, an input unit 16, a display unit 18, and an output unit 19.

The data acquisition unit 10 obtains dental image data. The data acquisition unit 10 may acquire dental image data through a linked medical imaging device (not shown) or a separate database, etc. The dental image data may include CT data, scan data, and the like.

The input unit 16 receives a user operation signal. At this time, the input unit 16 may receive a user operation signal for a user interface displayed on a screen.

The display unit 18 displays the results processed by the control unit 14 on the screen.

The output unit 19 may output a virtual implant surgical guide shape designed by the control unit 14 using a three-dimensional (3D) printer or milling device. Here, the designed virtual implant surgical guide shape may in a data format compatible with the 3D printer or milling device, and the 3D printer or milling device may output an actual implant surgical guide based on the input virtual implant surgical guide shape.

The storage unit 12 stores various types of data, including information necessary for operating the device 1 for designing an implant surgical guide and information generated during operation. For example, the storage unit 12 may store dental image data for individual patients and provide the data to the control unit 14 upon user request.

The control unit 14 establishes an implant surgery plan from dental image data and places a virtual object into the dental image data according to the established surgery plan. Examples of an object include crowns, implants, abutments, and the like.

According to an embodiment, the control unit 14 generates an arch line from the dental image data and generates a panoramic image using the generated arch line. Examples of arch line generation and panoramic generation will be described below with reference to FIGS. 2 to 15.

Next, the control unit 14 provides a virtual bone reduction simulation. For example, the control unit 14 displays a bone reduction interface for adjusting an amount of bone reduction in a panoramic image through the display unit 18. The bone reduction interface may be displayed in the form of a line or as a cross-sectional shape perpendicular to a plane. The control unit 14 may receive a user operation signal for the bone reduction interface through the input unit 16, and may adjust the amount of bone reduction. In this case, the control unit 14 may adjust the amount of bone reduction by vertically moving the position of the bone reduction interface according to the user operation signal for the bone reduction interface.

The control unit 14 may limit the movable range of the bone reduction interface. For example, the movable range of the bone reduction interface may be within a range that does not invade at least one of anatomical structures among the nerve canal, maxillary sinus, maxilla, and mandible.

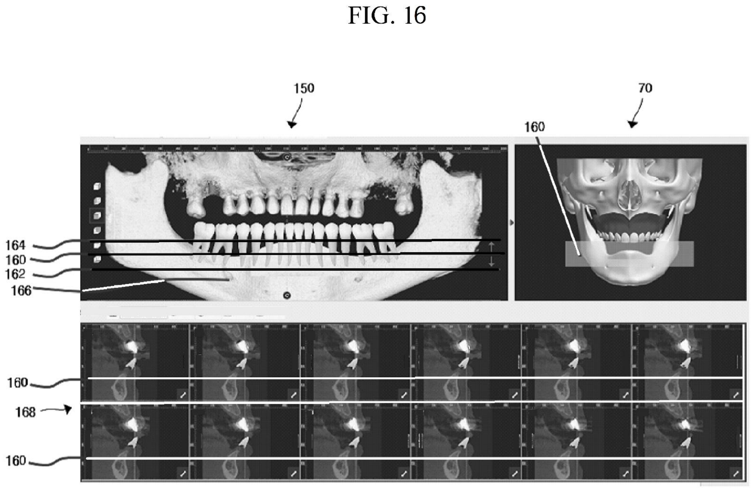

The control unit 14 may reduce virtual bone according to bone reduction adjustment through the bone reduction interface and display the result of the reduction through the display unit 18. An embodiment of bone reduction simulation will be described below with reference to FIG. 16.

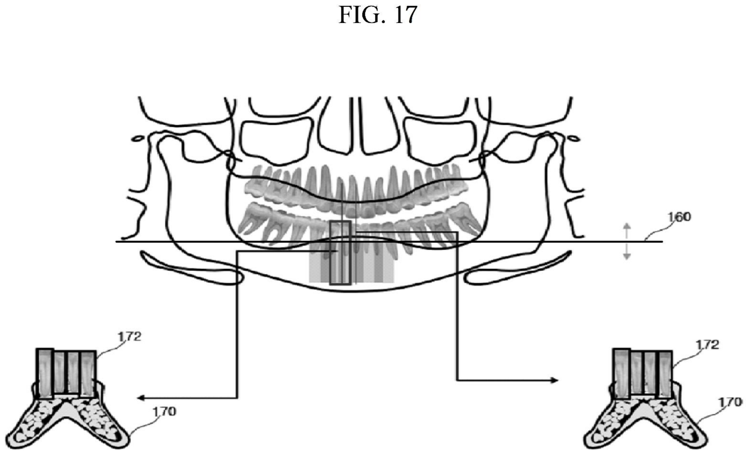

Subsequently, the control unit 14 may provide an implant placement simulation for placing a virtual implant according to virtual bone reduction performed through the bone reduction simulation. In this case, data generated according to the virtual bone reduction through the bone reduction simulation is defined as second CT data. An embodiment of the virtual implant placement simulation will be described below with reference to FIGS. 17 to 25.

Subsequently, the control unit 14 designs a virtual implant surgical guide shape and a virtual bone reduction guide shape from dental image data, for example, from the second CT data from which virtual bone has been reduced. The virtual implant surgical guide shape and virtual bone reduction guide shape designed through the control unit 14 may be output as a 3D actual implant surgical guide and actual implant surgical guide through the output unit 19. The actual implant surgical guide is actually fastened in the patient's oral cavity for implant surgery of the patient and guides the implant surgery. The actual bone reduction guide is actually fastened to the bone of the patient for bone reduction required during implant surgery of the patient and guides the bone reduction.

The virtual implant surgical guide shape and the virtual bone reduction guide shape generated by the control unit 14 may be displayed on the screen of the display unit 18.

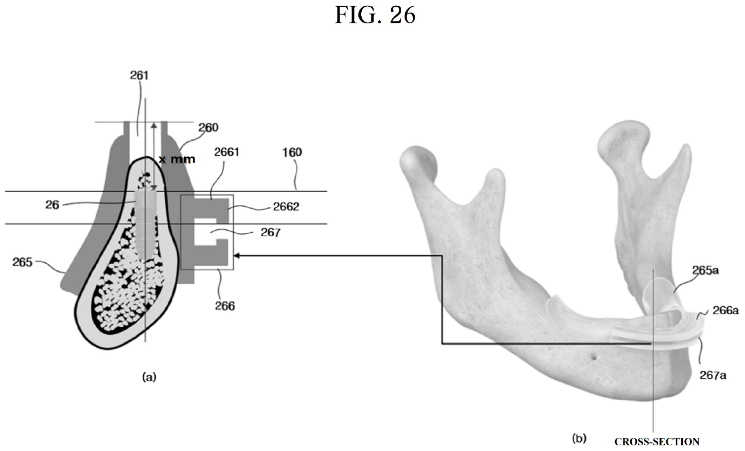

An embodiment of designing the virtual implant surgical guide shape and the virtual bone reduction guide shape will be described below with reference to FIGS. 26 to 32.

Meanwhile, the control unit 14 may design the virtual implant surgical guide shape and the virtual bone reduction guide shape in an integrated manner. In the case of integration, only a single guide needs to be manufactured, which simplifies the manufacturing process and reduces costs compared to manufacturing them separately.

However, depending on the patient's condition or the surgical tools provided, an integrated guide cannot be used in every case. Accordingly, the control unit 14 according to an embodiment may determine whether to integrate the virtual bone reduction guide shape and the virtual implant surgical guide shape, according to the bone height at the position of a placed virtual implant and the length of the guide drill, and notify the user of the result.

For example, when the bone height at the position of the placed virtual implant exceeds the length of the guide drill, the control unit 14 may recommend separate generation of the virtual bone reduction guide shape and the virtual implant surgical guide shape. In contrast, when the bone height at the position of the placed virtual implant does not exceed the length of the guide drill, the control unit 14 may recommend the integrated generation of the virtual bone reduction guide shape and the virtual implant surgical guide shape.

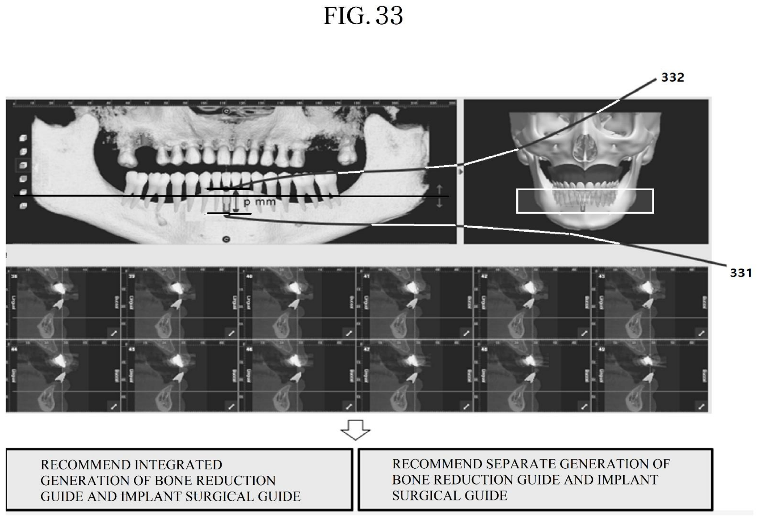

The process of integrated generation and separate generation of the virtual bone reduction guide shape and the virtual implant surgical guide shape of the device 1 for designing an implant surgical guide will be described below with reference to FIGS. 33 to 36.

FIG. 2 is a view illustrating dental image data according to an embodiment of the present invention.

Referring to FIGS. 1 and 2, the device 1 for designing an implant surgical guide may obtain CT data 21 and scan data 22 as dental image data of a patient. In this case, the patient may be an edentulous patient or a partially edentulous patient. The device 1 for designing an implant surgical guide may request and obtain the patient's dental image data from an external device. The scan data 22 may be data obtained using an intraoral scanner, and may be data obtained by taking an impression using an impression material and then scanning the impression, or data obtained by scanning a gypsum model produced by pouring gypsum into the impression.

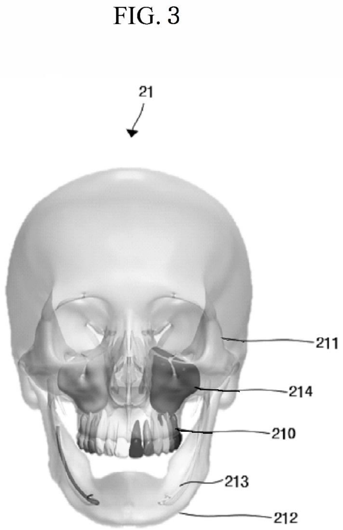

FIG. 3 is a view illustrating a screen for separating anatomical structures from dental image data according to an embodiment of the present invention.

Referring to FIGS. 1 and 3, the device 1 for designing an implant surgical guide may segment anatomical structures from dental image data, for example, first CT data 21. The device 1 for designing an implant surgical guide may segment the anatomical structures through an artificial neural network of artificial intelligence. For example, the device 1 for designing an implant surgical guide may learn anatomical structures of patients by training on CT data of previous patients, and then segment the anatomical structures using the training results. The anatomical structures may include maxillary teeth 210, mandibular teeth (not shown), a maxilla 211, a mandible 212, a nerve canal 213, maxillary sinus 214, and the like.

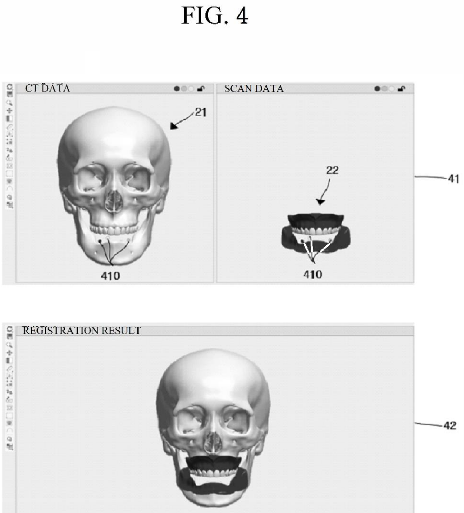

FIG. 4 is a view illustrating a screen for registering first CT data and scan data of a patient according to an embodiment of the present invention.

Referring to FIGS. 1 and 4, the device 1 for designing an implant surgical guide may register first CT data 21 and scan data 22 of a patient, which have different coordinate information. In the case of an edentulous patient, the device 1 for designing an implant surgical guide may generate a registration point at a predetermined position (e.g., a resin marker) and perform registration between two sets of data 21 and 22. In contrast, in the case of a partially edentulous patient having some remaining teeth, the device 1 for designing an implant surgical guide may register two sets of data 21 and 22 using feature points of the teeth. For example, the device 1 for designing an implant surgical guide may allow manual registration based on selection, made in response to a user operation signal, of one point or three points 410. Reference numeral 41 in FIG. 4 denotes a screen showing the registration process, and reference numeral 42 denotes a screen showing the result after registration is completed. In this case, the registered data of the first CT data 21 and the scan data 22 is referred to as "first registered data."

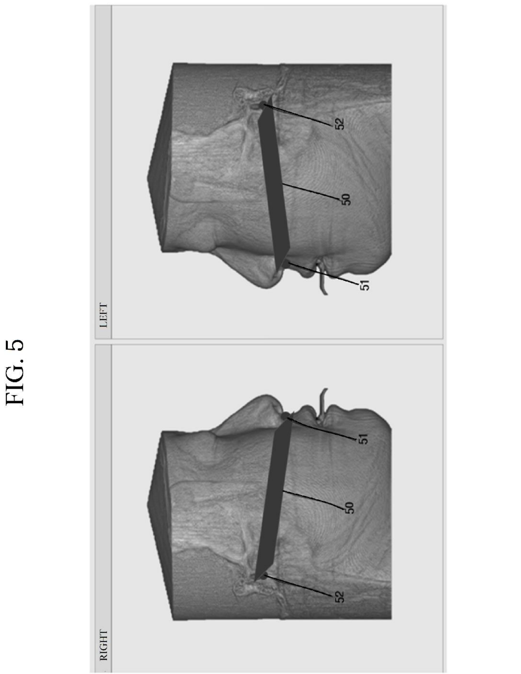

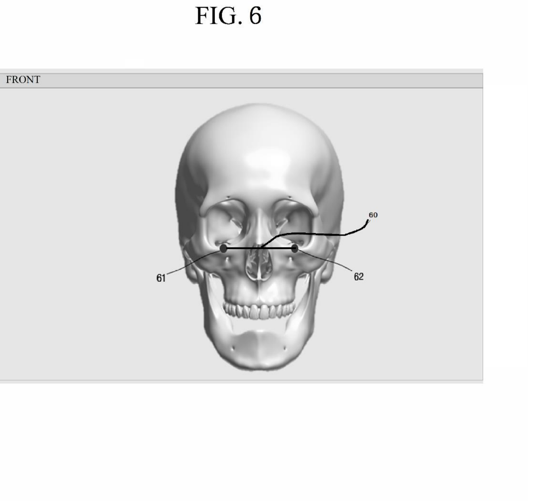

FIGS. 5 and 6 are views illustrating screens for setting an occlusal plane using the first CT data of the first registered data according to an embodiment of the present invention.

Referring to FIGS. 1, 5 and 6, the device 1 for designing an implant surgical guide resets the screen by providing axis information such that the orbits on both sides are displayed as one in a cross view of the first CT data of the first registered data, as shown in FIG. 5. The reason for resetting such that the orbits are displayed as one is to establish the occlusal plane in a complete lateral view.

As shown in FIG. 5, the device 1 for designing an implant surgical guide may set an occlusal plane 50 through user manipulation. To this end, the device 1 for designing an implant surgical guide adjusts brightness and contrast ratio of the first CT data of the first registered data so that soft tissue image appears. Subsequently, the device 1 for designing an implant surgical guide provides an interface that allows the user to select an inferior point 51 of the ala of the nose and a superior margin 52 of the tragus of the ear from each of the left and right sides of the first CT data of the first registered data. When the inferior point 51 of the ala of the nose and the superior margin 52 of the tragus are selected through user manipulation, the device 1 for designing an implant surgical guide sets the occlusal plane 50 by connecting the selected inferior point 51 of the ala of the nose and superior margin 52 of the tragus. In another example, the device 1 for designing an implant surgical guide may set the occlusal plane 50 by using artificial intelligence to learn the inferior point 51 of the ala of the nose and the superior margin 52 of the tragus.

As shown in FIG. 6, the device 1 for designing an implant surgical guide may automatically reset the screen by providing axis information through a line 60 connecting infraorbital margins 61 and 62 on both sides to be parallel in the coronal view.

As described above with reference to FIGS. 5 and 6, as the device 1 for designing an implant surgical guide resets the screen by providing axial information in the cross view and coronal view, the line 60 connecting the infraorbital margins 61 and 62 on both sides may be aligned parallel to the occlusal plane 50 of FIG. 5.

The device 1 for designing an implant surgical guide may set an arch line using the occlusal plane. The reason for setting the occlusal plane is that a virtual crown needs to be arranged to place a virtual implant, and the virtual implant must be placed perpendicular to the occlusal plane of the crown so that, when the actual implant is placed in the patient later, it can withstand vertically applied forces such as masticatory pressure.

An example of setting the infraorbital margins and occlusal plane has been described with reference to FIGS. 5 and 6. However, the reference line may include, in addition to this example, the Frankfort horizontal plane, and the like. The Frankfurt plane refers to a horizontal plane determined laterally between the lowest point of the orbital margin and the highest point of the external auditory meatus.

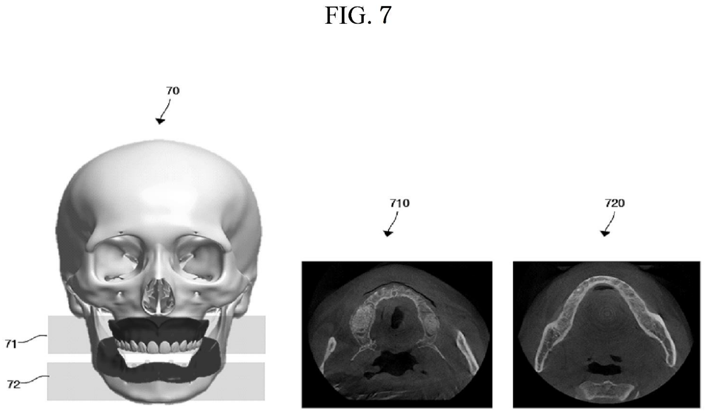

FIGS. 7 to 10 are views illustrating an arch line generation screen according to an embodiment of the present invention.

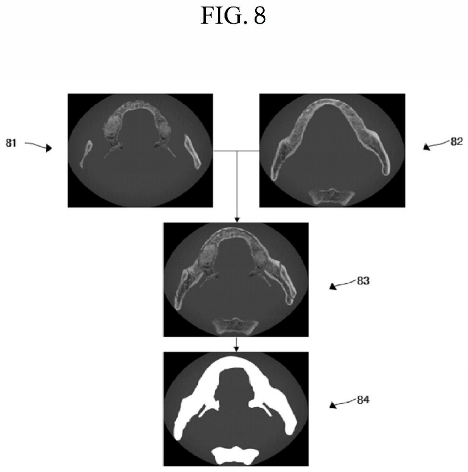

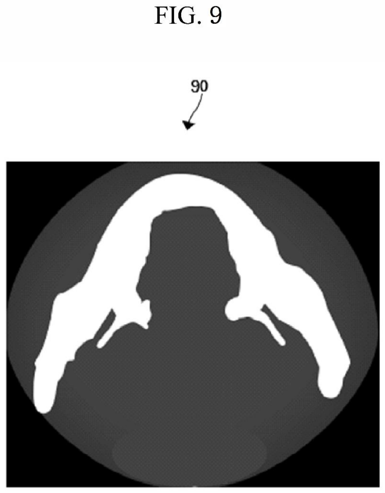

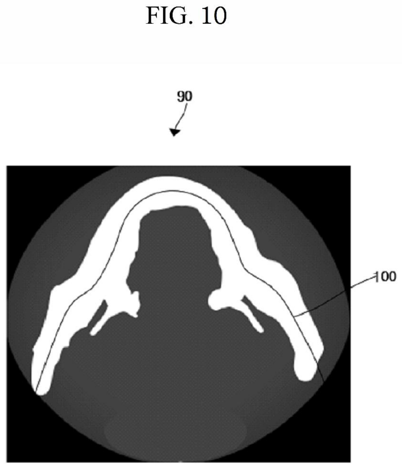

More specifically, FIG. 7 is a view illustrating a screen for separating the maxilla and mandible and extracting their respective images. FIG. 8 is a view illustrating a screen for generating a maximum intensity projection image. FIG. 9 is a view illustrating a screen for extracting the largest region among hard tissues from the maximum intensity projection image. FIG. 10 is a view illustrating a screen for generating an arch line in the maximum intensity projection image from which the largest region among hard tissues has been extracted.

Referring to FIGS. 1, 7 to 10, the device 1 for designing an implant surgical guide may generate an arch line using the first registered data obtained by registering the first CT data and the scan data. At this time, in the case of a partially edentulous patient, tooth information of the corresponding tooth may be used. For example, the device 1 for designing an implant surgical guide segments the teeth and forms a hexahedron in 3D and a rectangle in 2D for the segmented tooth. Subsequently, the device 1 for designing an implant surgical guide determines a point passing through a predetermined point (e.g., the center) of the hexahedron or rectangle as the tooth axis. The device 1 for designing an implant surgical guide uses the determined tooth axis to generate the arch line. In contrast, a maximum intensity projection image may be generated in an axial view to generate points for generating the arch line in a region where teeth are missing.

Hereinafter, the process of generating the arch line will be described in more detail.

As shown in FIG. 7, the device 1 for designing an implant surgical guide may extract a maxillary image 710 and a mandibular image 720 by separating a maxillary plane 71 and a mandibular plane 72 from the first registered data 70.

Subsequently, as shown in FIG. 8, the device 1 for designing an implant surgical guide extracts hard tissue from the maxillary image 710 and the mandibular image 720 of FIG. 7 and generate hard tissue images 81 and 82. For example, the device 1 for designing an implant surgical guide extracts hard tissues set to a brightness value of N (e.g., 255) from the maxillary image 710 and the mandibular image 720. The hard tissues may include teeth, bone, implants, and the like.

Subsequently, the device 1 for designing an implant surgical guide generates a combined image 83 by accumulating the maxillary image and mandibular image that are composed of the extracted hard tissue.

Next, the device 1 for designing an implant surgical guide generates a maximum intensity projection image 84, which is a 2D representation of the accumulated image 83. The maximum intensity projection image 84 is expressed as a 2D image in which cross-sections from the bone level of each of the maxilla and mandible to the lowest bone level, with respect to the occlusal plane, are combined. Accordingly, 3D data can be expressed in 2D through the maximum intensity projection image 84.

Then, the device 1 for designing an implant surgical guide generates an image 90 by extracting the largest region among the hard tissues from the maximum intensity projection image 84, as shown in FIG. 9. Through this, the tooth portion and jawbone among the hard tissues remain, while the cervical spine portion is removed.

Next, the device 1 for designing an implant surgical guide generates an arch line 100 in the maximum intensity projection image 90, from which the largest region among the hard tissues, as shown in FIG. 10. For example, the device 1 may generate the arch line 100 at the center based on one-half of the bone width in the maximum intensity projection image 90. Once the arch line 100 is generated, the shape of the arch line 100 can be viewed on the previously set occlusal plane. When the maximum intensity projection image is generated in the axial direction, the maxilla and mandible appear together as if in a single plane, so that the images of the maxilla and mandible overlap, resulting in a single arch line 100 being generated.

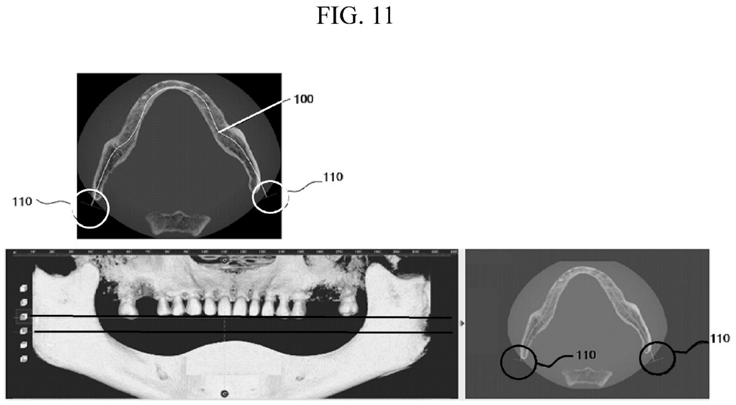

FIGS. 11 to 15 are views illustrating a virtual crown arrangement process according to an embodiment of the present invention.

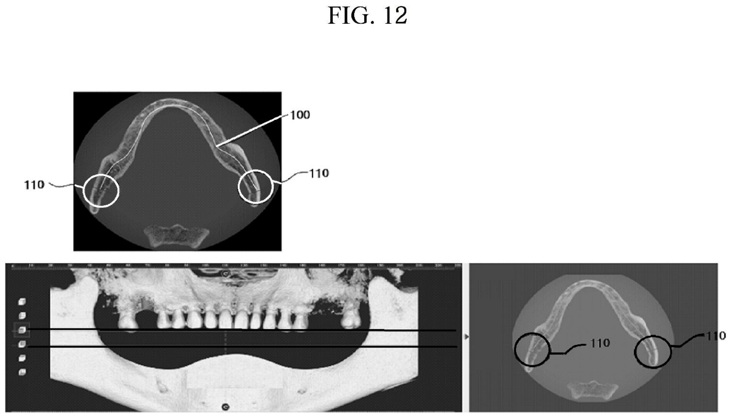

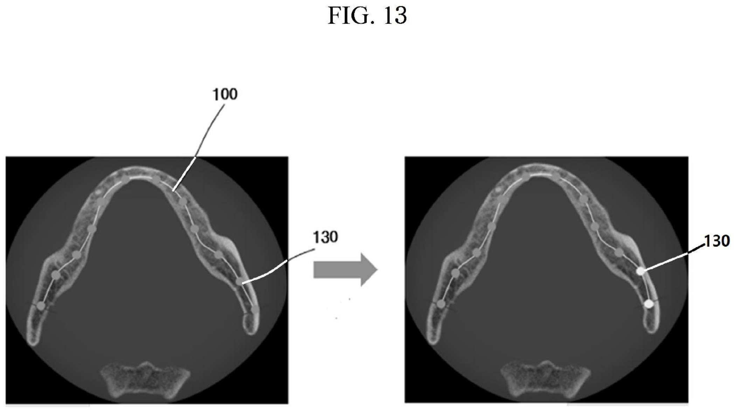

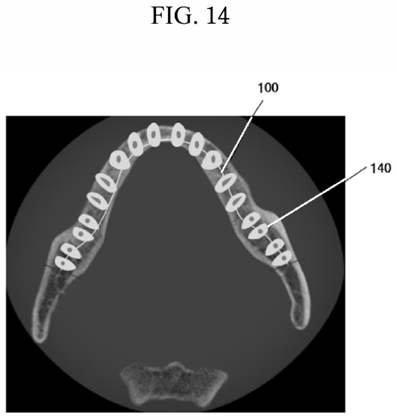

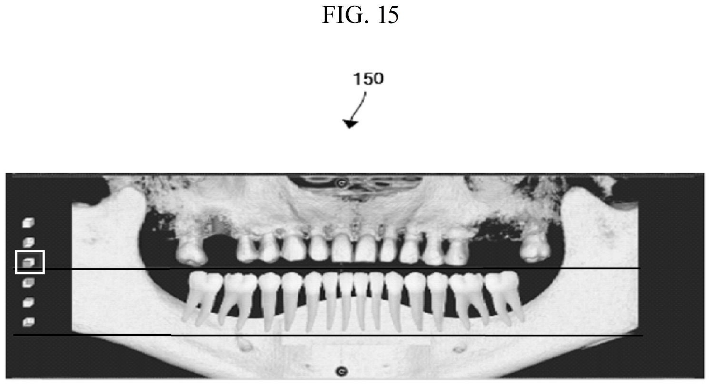

More specifically, FIG. 11 is a view illustrating a screen for setting the length of an arch line using the arch line length setting interface. FIG. 12 is a view illustrating a screen in which the length of the arch line is set. FIG. 13 is a view illustrating an arch line shape modification screen. FIG. 14 is a view illustrating a screen in which virtual crowns are arranged according to the length of the arch line. FIG. 15 is a view illustrating a screen for generating a panoramic image using the arch line.

Referring to FIGS. 1 and 11 to 15, the device 1 for designing an implant surgical guide may arrange virtual crowns based on the arch line. The process of crown arrangement will be described below.

As shown in FIG. 11, the device 1 for designing an implant surgical guide provides an arch line length setting interface 110, by which the length of the arch line 100 is set according to a user operation signal. The arch line length setting interface 110 is a user interface that allows the length of the arch line 100 to be adjusted by setting the boundary of the most posterior tooth (e.g., the distal end of the second molar) according to a user operation signal. FIG. 12 illustrates a screen in which the length of the arch line 100 is set through the arch line length setting interface 110.

Subsequently, the device 1 for designing an implant surgical guide may modify the shape of the arch line 100. For example, as illustrated in FIG. 13, the implant surgical guide design device 1 provides an arch line control point 130, which allows the user to modify the shape of the arch line 100. The user may modify the arch line shape by manipulating the arch line control point 130. The arch line control points 130 may be displayed at regular intervals by dividing the length of the arch line 100 into n equal parts. In the case of a partially edentulous patient, the device 1 for designing an implant surgical guide may form a hexahedron (or rectangle) on the segmented tooth as described above, and generate and display the arch line control point 130 based on a point passing through the center of the hexahedron (or rectangle). As the shape of the arch line 100 is modified by a user operation signal on the arch line control point 130, the shape of the panoramic image may also be changed in real time.

When the length and shape of the arch line 100 are set through the user interface, the device 1 for designing an implant surgical guide may arrange a virtual crown 140 on the arch line 100 by retrieving it from a library based on the set length of the arch line 100, as shown in FIG. 14. In this case, the device 1 for designing an implant surgical guide may arrange the crown 140 by enlarging or reducing the tooth shape through user manipulation according to the length of the arch line 100. In another example, the device 1 for designing an implant surgical guide may automatically arrange a virtual crown 140 of average size in proportion to the arch line 100. For example, when the length of the arch line 100 is 100 mm and the average size of the teeth is 80 mm, the virtual crown 140 may be automatically arranged to fit the 100 mm arch line 100. The virtual crown 140 arranged on the arch line 100 may subsequently be used as tooth axis information when placing a virtual implant.

Subsequently, the device 1 for designing an implant surgical guide may generate a panoramic image 150 based on the arch line, as shown in FIG. 15. The panoramic image 150 may be a 3D or 2D image. Here, when the panoramic image 150 is a 3D panoramic image, an additional Z-axis plane is present compared to a 2D panoramic image. Therefore, the 3D panoramic image can provide the user with visually advantageous information during bone reduction simulation and virtual implant placement so as to be parallel with to the occlusal plane.

FIG. 16 is a view illustrating a movable range of a bone reduction interface according to an embodiment of the present invention.

Referring to FIGS. 1 and 16, the device 1 for designing an implant surgical guide displays a bone reduction interface 160 for adjusting an amount of bone reduction on the panoramic image 150. The bone reduction interface 160 may be in the form of a cross-section perpendicular to a line or plane. The bone reduction interface 160 may be displayed so as to be parallel to the occlusal plane. The device 1 for designing an implant surgical guide adjusts the amount of bone reduction according to a user operation signal through the displayed bone reduction interface 160.

The device 1 for designing an implant surgical guide 1 may set a movable range of the bone reduction interface 160. The user may move the position of the bone reduction interface 160 (e.g., upward and downward) by a user operation signal within the set movable range. At this time, the movable range of the bone reduction interface 160 may be within a range that does not invade at least one of anatomical structures among the nerve canal, maxillary sinus, maxilla, and mandible. In the case of the mandible, an upper limit of the bone reduction interface 160 may be the uppermost position of a mandible line 164. Additionally, a lower limit of the bone reduction interface 160 may be a position 162 located at a predetermined distance (e.g., 2 mm) above the foramen 166 of the nerve canal. In the case of the maxillary sinus, the movable range of the bone reduction interface 160 may be up to a position before contacting the maxillary sinus or a position spaced apart by a predetermined distance.

The device 1 for designing an implant surgical guide may display the bone reduction interface 160 on a CT cross-sectional image 168. In another example, the device 1 for designing an implant surgical guide may also display the bone reduction interface 160 on the first registered data 70 in which the first CT data and scan data are registered.

When an amount of bone reduction is determined by user manipulation through the bone reduction interface 160, the device 1 for designing an implant surgical guide may display to the user a state in which the bone is reduced, on the panoramic image 150, the CT cross-sectional image 168, or the first registered data 70. In the case of CT cross-sectional image 168, the bone-reduced portion may be displayed as a line. Data in which second CT data in which bone is reduced and scan data are registered may be stored as separate second registered data. The second registered data may be used to generate a virtual implant guide shape and a bone reduction guide shape, which will be described below with reference to FIG. 26.

FIG. 17 is a view illustrating a screen for displaying, through a user interface, a permissible position for virtual implant placement, according to an embodiment of the present invention.

Referring to FIGS. 1 and 17, when virtual bone reduction is performed, the device 1 for designing an implant surgical guide may form a cross-section perpendicular to the line or plane of the bone reduction interface 160. The device 1 for designing an implant surgical guide may detect a bone margin 170 of the cross-section using an HU value in the second CT data. At this time, the device 1 for designing an implant surgical guide determines a point where the HU value sharply increases as cortical bone, and by connecting this boundary line, may detect the bone margin 170 on the corresponding cross-section.

The device 1 for designing an implant surgical guide may display a permissible position for virtual implant placement according to a line or plane of the bone reduction interface 160, the position of which is adjusted (e.g., upward or downward) in accordance with a user operation signal through the bone reduction guide interface 160.

The device 1 for designing an implant surgical guide may form a tooth axis based on remaining teeth or arranged virtual teeth, and may place a virtual implant along the tooth axis. Here, the tooth axis may be displayed as a line passing through the center of a hexahedron (3D) or a rectangle (2D) 172.

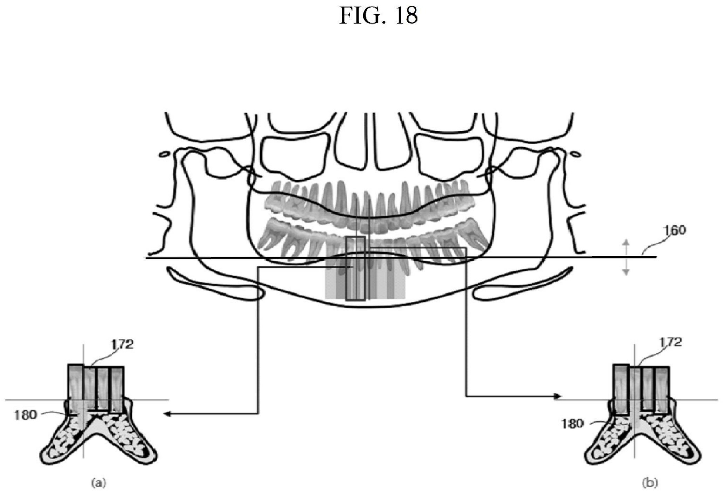

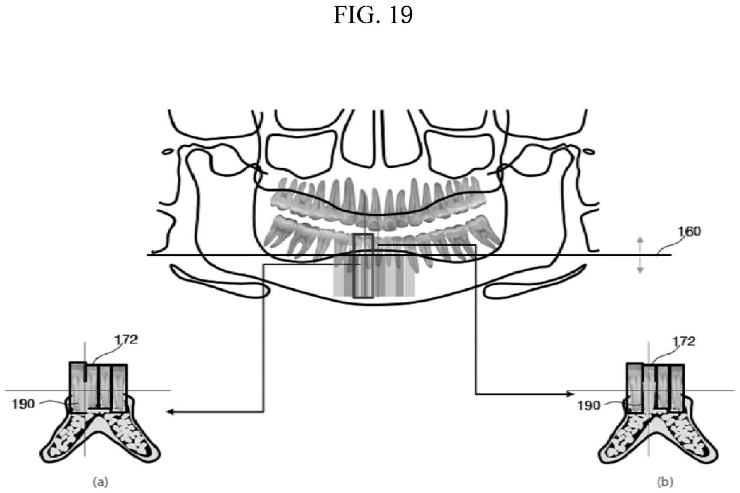

FIG. 18 and FIG. 19 are views illustrating a screen for placing a virtual implant according to an embodiment of the present invention.

More specifically, FIG. 18 is a view illustrating a screen for placing a submerged type implant, and FIG. 19 is a view illustrating a screen for placing a non-submerged type implant. The submerged type implant refers to an implant at the bone level, and the non-submerged type implant refers to an implant at the tissue level.

Referring to FIGS. 1, 18, and 19, the device 1 for designing an implant surgical guide may divide the diameter of virtual implants 180 and 190 to be placed in half with respect to the central axis of the tooth axis and place the virtual implants 180 and 190 at the center of the tooth axis.

(a) and (b) in FIGS. 18 and 19 are coronal views, where (a) shows the virtual implants 180 and 190 placed in a state deviating from the tooth axis, while (b) shows the virtual implants 180 and 190 placed in alignment with the tooth axis 172.

The device 1 for designing an implant surgical guide may vary the placement position according to the type of virtual implant to be placed. For example, as shown in FIG. 18, when the virtual implant to be placed is a submerged type implant, the virtual implant 180 may be placed at a predetermined distance (e.g., 1 mm) below the line (on a 2D cross-section) or plane (in 3D) of the bone reduction interface 160. In contrast, as shown in FIG. 19, when the virtual implant to be placed is a non-submerged type implant, the device 1 for designing an implant surgical guide may place the virtual implant 190 so as to be aligned with the line (on a 2D cross-section) or plane (in 3D) of the bone resection interface 160.

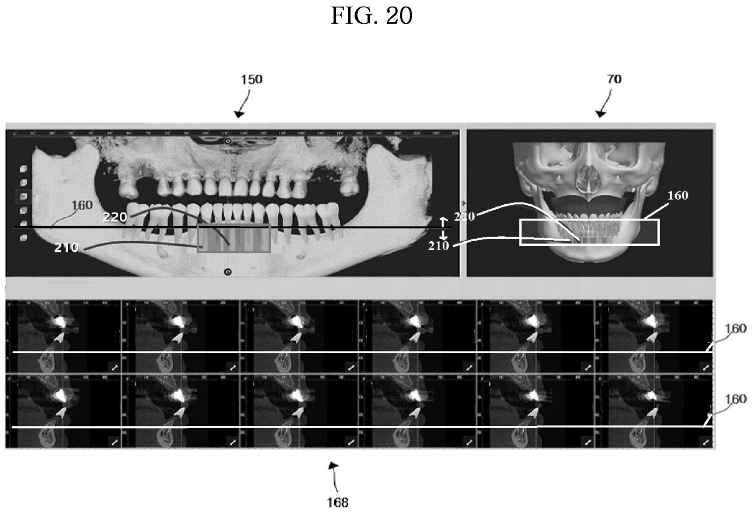

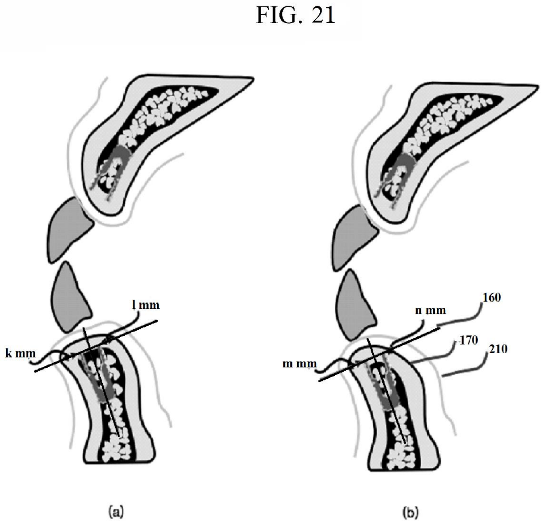

FIG. 20 is a view illustrating a screen for displaying, in a distinguishable manner, a permissible position for virtual implant placement and a non-permissible position for virtual implant placement, according to an embodiment of the present invention, and FIG. 21 is a view illustrating a bone width that serves as a criterion for distinguishing between a permissible position for virtual implant placement and a non-permissible position for virtual implant placement according to an embodiment of the present invention.

Referring to FIGS. 1, 20, and 21, when placing a virtual implant in the panoramic image 150, the CT cross-sectional image 168, or the first registered data 70, if a bone width from the top end portion of the virtual implant is not secured to be greater than or equal to a predetermined distance (e.g., 2 mm) (FIG. 21(b)), the device 1 for designing an implant surgical guide may display the position as a non-permissible position 210 for virtual implant placement. At this time, the non-permissible position 210 for virtual implant placement may be displayed in a predetermined color (e.g., blue) so that the user can distinguish it. In FIG. 21(b), both m mm and n mm are less than or equal to the predetermined distance (e.g., 2 mm).

In comparison, when the bone width from the top end portion of the virtual implant is secured to be greater than or equal to a predetermined distance (e.g., 2 mm) (FIG. 21(a)), the device 1 for designing an implant surgical guide may display the position as a permissible position 220 for virtual implant placement. At this time, the permissible position 220 for virtual implant placement may be displayed in a predetermined color (e.g., red) so that the user can distinguish it. In (a) of FIG. 21, k mm and 1 mm are both equal to or greater than the predetermined distance (e.g., 2 mm).

The device 1 for designing an implant surgical guide may distinguish the colors of the non-permissible position 210 for virtual implant placement and the permissible position 220 for virtual implant placement by using predetermined colors or by using colors set by the user. Furthermore, in addition to color, various forms such as patterns, shading, and the like may be used for distinction.

In FIG. 21, reference numeral 160 denotes a line or plane of the bone reduction interface, reference numeral 170 denotes a bone margin, and reference numeral 210 denotes a gingival line after registration.

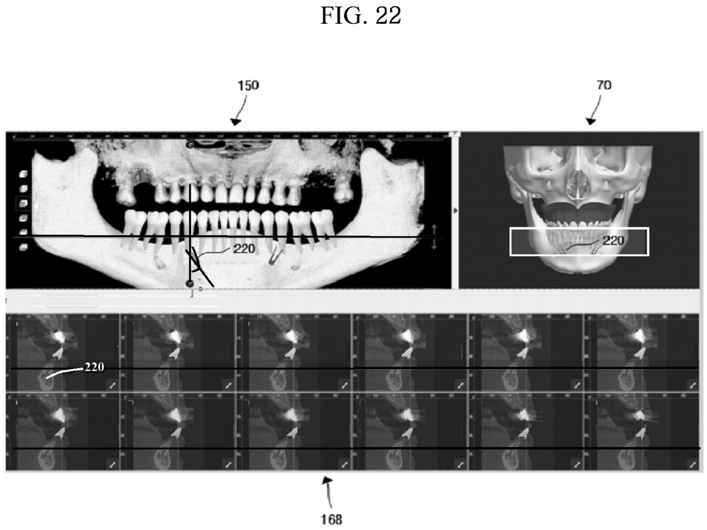

FIG. 22 is a view illustrating a screen for placing a virtual implant inclined at a predetermined angle with respect to the tooth axis according to an embodiment of the present invention.

Referring to FIGS. 1 and 22, the device 1 for designing an implant surgical guide may display a virtual implant placement position on a cross-sectional surface perpendicular to the occlusal plane within a range where bone reduction has been performed in the panoramic image 150, the CT cross-sectional image 168, or the first registered data 70. In this case, the device 1 for designing an implant surgical guide may place a virtual implant 220 in a shape inclined at a predetermined angle j (for example, within a range of 17 degrees to 30 degrees) with respect to the tooth axis.

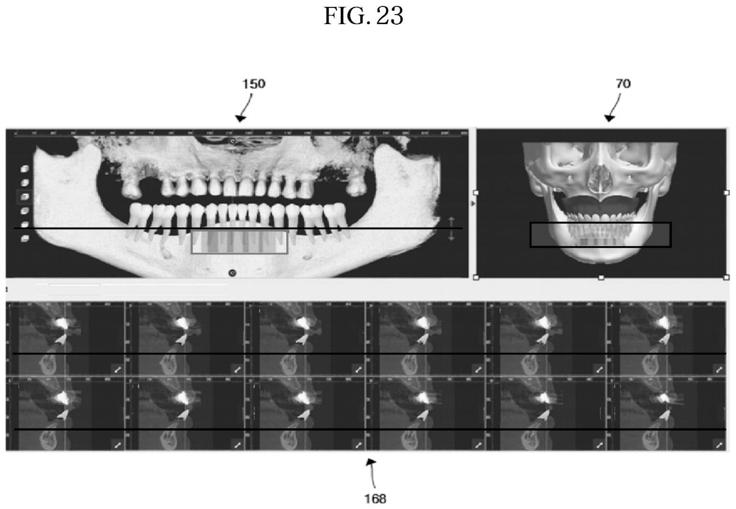

FIG. 23 is a view illustrating a screen for displaying a permissible placement position of a virtual implant having a corresponding diameter, according to a change in the diameter of a virtual implant by a user, according to an embodiment of the present invention.

Referring to FIGS. 1 and 23, the device 1 for designing an implant surgical guide may provide the user with an implant diameter adjustment interface (not shown) for changing the diameter of a virtual implant. When the user changes the diameter of the virtual implant through the implant diameter adjustment interface (not shown), the device 1 for designing an implant surgical guide may display, on the panoramic image 150, the CT cross-sectional image 168, or the first registered data 70, a permissible placement position of the virtual implant having the changed diameter. For example, depending on the user's change of the virtual implant diameter, the screen of FIG. 20 may be changed to that shown in FIG. 23. The length of the virtual implant to be placed and the gingival height (only in the case of a non-submerged type implant) are preset according to the dental formula. When there is contact with an anatomical structure such as the maxillary sinus or the nerve canal, the device 1 for designing an implant surgical guide may identify the corresponding virtual implant through a collision algorithm and may issue a warning to the user so that the user can recognize it. For example, the corresponding virtual implant may be displayed in a distinguishable color.

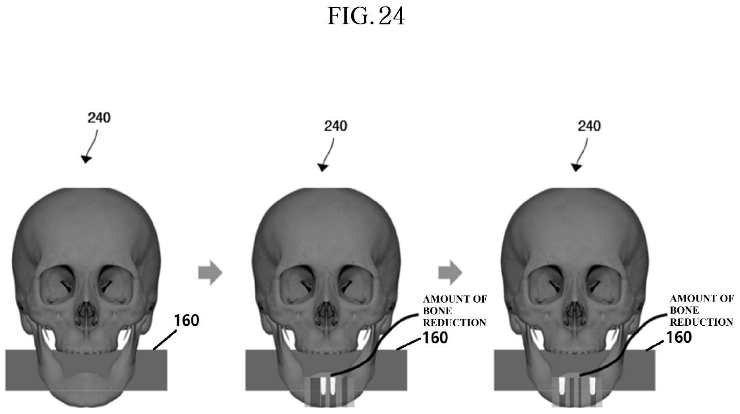

FIG. 24 is a view illustrating a screen for displaying a change in an implant placement position when a user changes the diameter of a virtual implant to be placed, according to an embodiment of the present invention.

Referring to FIGS. 1 and 24, the device 1 for designing an implant surgical guide may, after adjusting an amount of bone reduction through the bone reduction interface 160, display a permissible placement position of a virtual implant having a changed diameter according to the user's change of the virtual implant diameter made through the implant diameter adjustment interface (not shown). For example, as shown in FIG. 24, when the user changes the diameter of the virtual implant to be placed from (a) the initial diameter to (b) 3.0 mm, and further to (c) 3.5 mm, the virtual implant placement position may be changed according to each changed diameter and displayed in the panoramic image (not shown), the first registered data (not shown), the first CT data 240 of the first registered data, or the CT cross-sectional image (not shown).

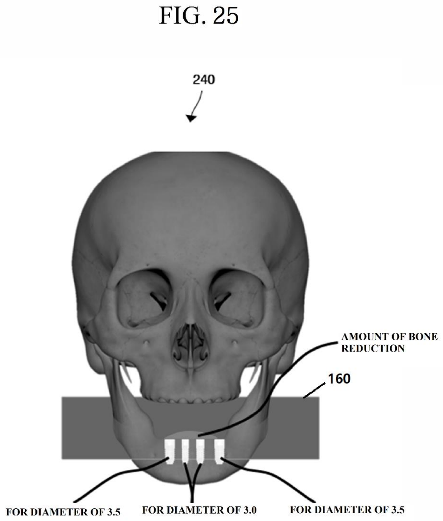

FIG. 25 is a view illustrating a screen displaying all virtual implant placement positions selected by the user, according to an embodiment of the present invention.

Referring to FIGS. 1 and 25, the device 1 for designing an implant surgical guide may display, in a panoramic image (not shown), the first registered data (not shown), or the first CT data 240 of the first registered data, all of the virtual implant placement positions for each diameter selected according to the user's selection of the virtual implant diameter. The user may check the virtual implant placement positions by diameter in the image and select a desired virtual implant among them.

FIG. 26 is a view illustrating an example of generating a virtual implant surgical guide shape and a virtual bone reduction guide shape according to an embodiment of the present invention.

More specifically, (a) of FIG. 26 is a cross-sectional view of a structure of a virtual implant surgical guide shape and a virtual bone reduction guide shape, and (b) is a view illustrating a state in which a bone reduction guide manufactured according to the virtual bone reduction guide shape is fastened to first CT data. A vertical cross-section of FIG. 26(b) corresponds to FIG. 26(a).

Referring to FIGS. 1 and 26, when a virtual implant is placed, the device 1 for designing an implant surgical guide designs the virtual implant surgical guide shape and the virtual bone reduction guide shape on dental image data, for example, on second CT data in which a virtual bone has been reduced. The virtual implant surgical guide shape and the virtual bone reduction guide shape designed through the device 1 for designing an implant surgical guide may be output as a 3D actual implant surgical guide and an actual bone reduction guide. The implant surgical guide is actually fastened in the patient's oral cavity for implant surgery of the patient and guides the implant surgery. The bone reduction guide is actually fastened to the patient's bone for bone reduction required during implant surgery of the patient and guides the bone reduction.

Hereinafter, with reference to FIG. 26, the process of generating the virtual implant surgical guide shape and the virtual bone reduction guide shape will be described.

The implant surgical guide design device 1 generates a virtual implant surgical guide shape 260 including a virtual implant guide hole 261 from dental image data, for example, from second CT data in which virtual bone has been reduced. The virtual implant guide hole 261 may be generated at a position spaced apart by x mm from the uppermost height of a placed virtual implant 26. The reason for the x-mm spacing is that a length of contact must be ensured between a guide portion of the implant surgical guide capable of guiding a drill and a guide portion of the drill. At this time, the device 1 for designing an implant surgical guide may generate the virtual implant surgical guide shape 260 such that the axis of the virtual implant 26 coincides with the center point of the virtual implant guide hole 261. The diameter of the virtual implant guide hole 261 and the spacing length x mm from the uppermost end of the virtual implant 26 may vary depending on the manufacturer.

Subsequently, the device 1 for designing an implant surgical guide generates a virtual bone reduction guide shape 265 that guides bone reduction parallel to the occlusal plane. At this time, the device 1 may generate an outermost contour line of the virtual bone reduction guide shape 265 on the basis of the position of the virtual implant guide hole 261. In addition, the device 1 for designing an implant surgical guide may generate the outermost contour line of the virtual bone reduction guide shape 265 on the basis of the line 160 or plane of the bone reduction interface whose position is adjusted (for example, upward or downward) in accordance with a user operation signal.

The device 1 for designing an implant surgical guide may also generate the virtual bone reduction guide shape 265 through user manipulation. For example, the device 1 for designing an implant surgical guide may generate points in response to a user operation signal and connect the generated points to generate the outermost contour line of the virtual bone reduction guide shape 265. In another example, the device 1 for designing an implant surgical guide may generate the outermost contour line of the virtual bone reduction guide shape 265 by allowing the user to directly draw a line through a user operation signal.

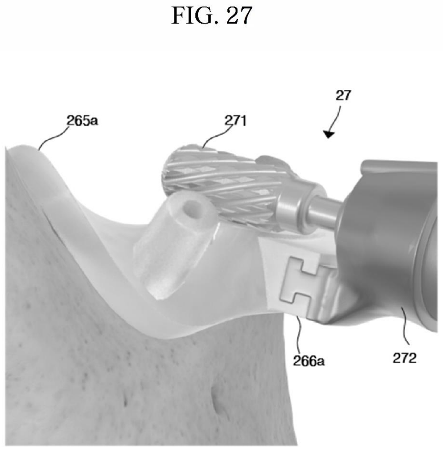

Subsequently, the device 1 for designing an implant surgical guide may generate a virtual guide rail 266 that protrudes along the outer periphery of the virtual bone reduction guide shape 265 while including a virtual insertion portion 267. At this time, an actual guide rail 266a includes an actual insertion portion 267a into which a guiding mechanism 272 of a bone reduction device 27 (see FIG. 27) is inserted. The guiding mechanism 272 (in FIG. 272) inserted into the actual insertion portion 267a is movable along the outer periphery of the actual bone reduction guide 265a. When the guiding mechanism 272 (in FIG. 27) moves along a movement path restricted by the actual guide rail 266a, a cutting mechanism 271 coupled to the guiding mechanism 272 may move together and reduce bone protruding above the opening of the actual bone reduction guide.

The virtual guide rail 266 may be generated within the movable range of the bone reduction interface 160. The movable range of the bone reduction interface 160 may be within a range that does not invade at least one of anatomical structures among the nerve canal, maxillary sinus, maxilla, and mandible.

By generating a line or plane of the bone reduction interface 160 parallel to the occlusal plane and by generating a plane 2661 of the virtual guide rail 266 parallel to the line or plane of the bone reduction interface 160, the virtual guide rail 266 may be generated parallel to the occlusal plane. For example, as shown in FIG. 26(a), the virtual guide rail 266 has the plane 2661 and a vertical surface 2662, where the plane 2661 may be parallel to the occlusal plane and the vertical surface 2662 may be perpendicular to the occlusal plane. The plane 2661 is parallel to the occlusal plane, and the vertical surface 2662 is a surface to which the guiding mechanism 272 of a bone reduction device (27 of FIG. 27) is coupled. Accordingly, the bone reduction device 27 (in FIG. 27) coupled to the actual guide rail 266a through the guiding mechanism 272 may reduce bone parallel to the occlusal plane. Furthermore, the virtual guide rail 266 may be formed at a position spaced apart by a predetermined distance vertically and horizontally from the line or plane of the bone reduction interface 160. For example, in the case of maxilla, the virtual guide rail 266 may be formed at a position spaced apart upward from the bone reduction interface 160 by a predetermined distance, and when in the case of mandible, the virtual guide rail 266 may be formed at a position spaced apart downward from the bone reduction interface 160 by a predetermined distance.

As shown in FIG. 26(b), the actual bone reduction guide 265a manufactured by outputting the virtual bone reduction guide shape 265 is mounted on the patient's bone. The actual bone reduction guide 265a may include an opening through which the patient's bone to be reduced protrudes. Subsequently, the bone reduction device may be mounted by the user onto the actual guide rail 266a within the actual bone reduction guide 265a. Thereafter, the cutting mechanism 271 of the bone reduction device 27 (in FIG. 27) may be moved along the path of the actual guide rail 266a by the user, thereby reducing the bone protruding above the opening of the actual bone reduction guide 265a. The bone reduction device will be described below with reference to FIG. 27.

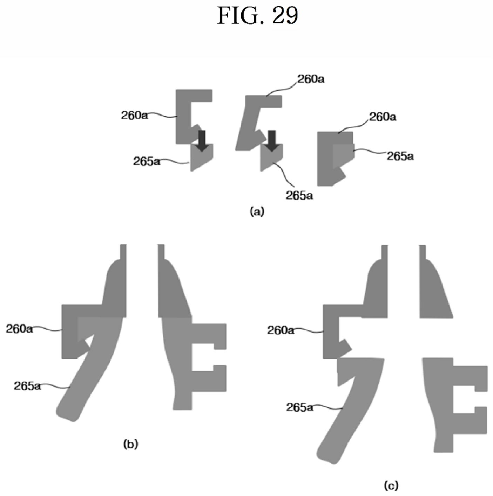

Subsequently, the device 1 for designing an implant surgical guide designs a coupled form of the virtual implant surgical guide shape and the virtual bone reduction guide shape. The design of the coupled form of the virtual implant surgical guide shape and the virtual bone reduction guide shape will be described below with reference to FIGS. 29 to 31.

FIG. 27 is a view illustrating an actual bone reduction device according to an embodiment of the present invention.

Referring to FIGS. 1 and 27, the actual bone reduction device 27 includes the cutting mechanism 271 and the guiding mechanism 272. The cutting mechanism 271 is a mechanism for reducing actual bone using a plurality of cutting blades. The guiding mechanism 272 is a mechanism that is coupled to each of the cutting mechanism 271 and the actual bone reduction guide 265a and supports them.

The actual bone reduction guide 265a is mounted onto the patient's actual bone by the user, and the guiding mechanism 272 of the bone reduction device 27 is mounted onto the actual guide rail 266a of the actual bone reduction guide 265a. Subsequently, the cutting mechanism 271 mounted to the guiding mechanism 272 by the user moves along the path of the guide rail 266a and reduces the bone protruding above the bone reduction guide 265a. The cutting mechanism 271 and the bone reduction guide 265a may be formed in a shape parallel to the occlusal plane or the bone reduction line 160 in FIG. 26 so that actual bone can be reduced.

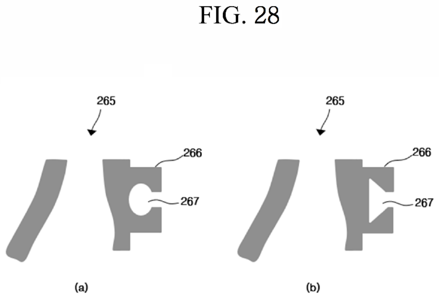

FIG. 28 is a view illustrating a virtual guide rail form of the virtual bone reduction guide shape according to an embodiment of the present invention.

Referring to FIGS. 27 and 28, the virtual guide rail 266 within the virtual bone reduction guide shape 265 includes an insertion portion 267 into which the guiding mechanism 272 of the bone reduction device 27 can be inserted. The guiding mechanism 272 is inserted into the insertion portion 267 and moves horizontally. The guide rail 266 has a form in which the guiding mechanism 272 and the occlusal plane are perpendicular to each other. The insertion portion 267 inside the guide rail 266 may take any form as long as the plane of the guide rail 266 is parallel to the occlusal plane and the vertical surface of the guide rail 266 is perpendicular to the occlusal plane. For example, the insertion portion 267 may be circular (a), triangular (b), or polygonal.

FIGS. 29 to 31 are views illustrating examples of designing a coupled form of a virtual implant placement guide shape and a virtual bone reduction guide shape according to various embodiments of the present invention.

Referring to FIGS. 1 and 29 to 31, the device 1 for designing an implant surgical guide designs a coupled form of a virtual implant placement guide shape and a virtual bone reduction guide shape in order to couple an implant surgical guide manufactured according to the virtual implant surgical guide shape and a bone reduction guide manufactured according to the virtual bone reduction guide shape.

For example, as shown in FIG. 29, the device 1 for designing an implant surgical guide designs the coupled form of the virtual implant surgical guide shape and the virtual bone reduction guide shape such that, when the actual implant surgical guide 260a is fastened to the actual bone reduction guide 265a, the actual implant surgical guide 260a is coupled in a manner where it is fitted into the actual bone reduction guide 265a by physical force. The materials of the actual implant surgical guide 260a and the actual bone reduction guide 265a have elasticity so that the actual implant surgical guide 260a can be snapped into the actual bone reduction guide 265a.

(a) of FIG. 29 is a view illustrating a process in which the actual implant surgical guide 260a is fitted into the actual bone reduction guide 265a, (b) is a view illustrating a state in which the actual implant surgical guide 260a is fitted into the actual bone reduction guide 265a, and (c) is a view illustrating a state before the actual implant surgical guide 260a is fitted into the actual bone reduction guide 265a.

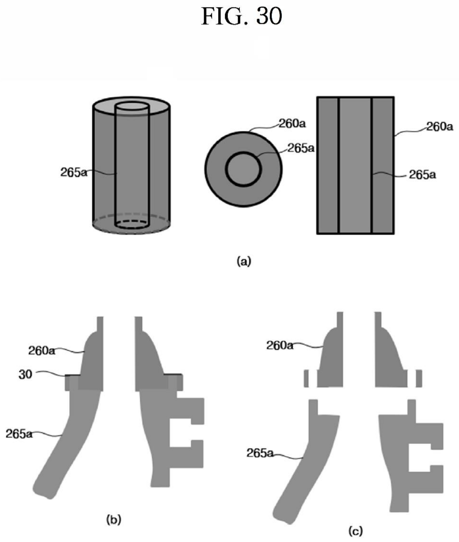

In another example, the device 1 for designing an implant surgical guide may design the coupled form of the virtual implant surgical guide shape and the virtual bone reduction guide shape such that the actual bone reduction guide 265a is mounted in a cylindrical groove of the actual implant surgical guide 260a, as shown in FIG. 30.

(a) of FIG. 30 is a three-dimensional illustration of the coupled form of the actual implant surgical guide 260a and the actual bone reduction guide 265a, (b) is a view illustrating a state in which the actual implant surgical guide 260a is coupled to the actual bone reduction guide 265a, and (c) is a view illustrating a state before the actual implant surgical guide 260a is coupled to the actual bone reduction guide 265a.

When the actual implant surgical guide 260a is precisely coupled to the actual bone reduction guide 265a, their heights coincide, as shown by reference numeral 30 in FIG. 30(b).

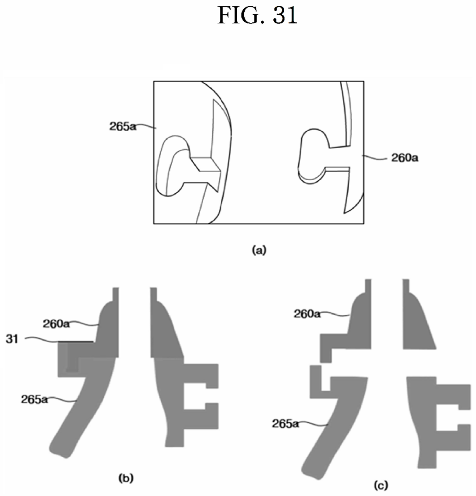

In another example, the device 1 for designing an implant surgical guide may design the coupled form of the virtual implant surgical guide shape and the virtual bone reduction guide shape such that the actual implant surgical guide 260a and the actual bone reduction guide 265a are coupled in an omega (Ω) shape, as shown in FIG. 31.

(a) of FIG. 30 is a three-dimensional illustration of the coupled form of the actual implant surgical guide 260a and the actual bone reduction guide 265a, (b) is a view illustrating a state in which the actual implant surgical guide 260a is coupled to the actual bone reduction guide 265a, and (c) is a view illustrating a state before the actual implant surgical guide 260a is coupled to the actual bone reduction guide 265a.

When the actual implant surgical guide 260a is precisely coupled to the actual bone reduction guide 265a, their heights coincide, as shown by reference numeral 31 in FIG. 30(b).

With reference to FIGS. 29 to 31, the positions of the implant surgical guide 260 and the bone reduction guide 265 described above may also be reversed.

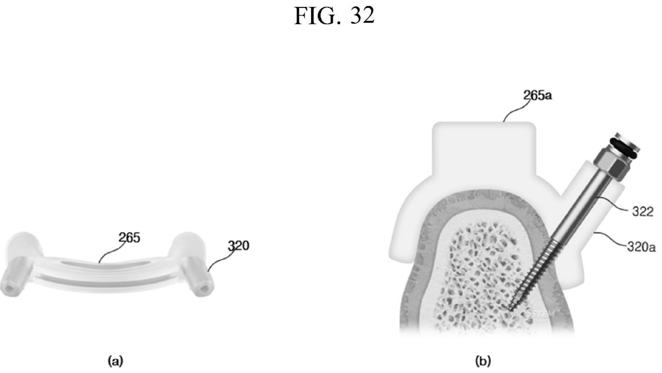

FIG. 32 is a view illustrating an example of FIG. 32 is a view illustrating an example of generating a virtual anchor pin drilling hole in the virtual bone reduction guide shape according to an embodiment of the present invention.

More specifically, (a) of FIG. 32 is a view illustrating a state in which a virtual anchor pin drilling hole 320 is generated in the virtual bone reduction guide shape 265, and (b) is a view illustrating a state in which an anchor pin 322 is inserted into an actual anchor pin drilling hole 320a of the actual bone reduction guide 265a manufactured according to the virtual bone reduction guide shape 265.

Referring to FIGS. 1 and 32, in the case of an edentulous patient having no teeth, the device 1 for designing an implant surgical guide may design a separate virtual anchor pin drilling hole 320 for fixing the anchor pin 322 to secure the bone reduction guide 265a to the bone so that it does not move.

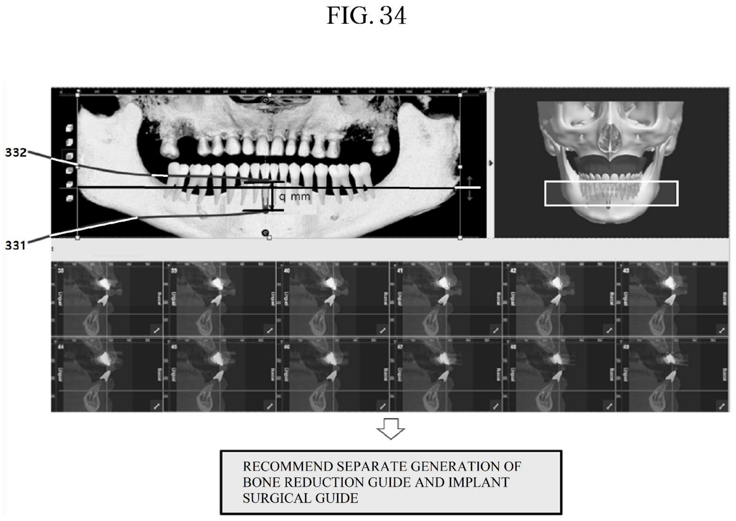

FIGS. 33 and 34 are views illustrating screens that recommend integrated generation or separate generation of a virtual bone reduction guide shape and a virtual implant surgical guide shape according to the length of a guide drill according to an embodiment of the present invention.

More specifically, FIG. 33 is a view illustrating a screen that recommends a selection between integrated generation and separate generation of a virtual bone reduction guide shape and a virtual implant surgical guide shape, and FIG. 34 is a view illustrating a screen that recommends separate generation of the virtual bone reduction guide shape and the virtual implant surgical guide shape.

Referring to FIGS. 1, 33, and 34, the device 1 for designing an implant surgical guide obtains length information of a guide drill for implant surgery, and determines whether to integrate the virtual bone reduction guide shape and the virtual implant surgical guide shape according to the length of the guide drill. In this case, when separation is determined, the device 1 for designing an implant surgical guide separately generates the virtual bone reduction guide shape and the virtual implant surgical guide shape from the dental image data. In contrast, when integration is determined, the device 1 generates the virtual bone reduction guide shape and the virtual implant surgical guide shape in an integrated manner from the dental image data.

In an embodiment, the device 1 for designing an implant surgical guide determines the bone height before bone reduction with respect to the position of the virtual implant placed in the dental image data in order to determine whether to or not to perform integration. In this case, the bone height may be a distance obtained by adding the distance from the lowermost position 331 of the virtual implant to the uppermost position 332 of the bone before bone reduction, with respect to the axis of the virtual implant.

Subsequently, the device 1 for designing an implant surgical guide checks the length of the guide drill. To this end, the device 1 receives a selection of a guided surgery kit that enables virtual implant placement according to a user operation signal. Next, the device 1 for designing an implant surgical guide may obtain information on the maximum length of the guide drill from a library according to the selected guided surgery kit.

Subsequently, the device 1 for designing an implant surgical guide compares the bone height and the length of the guide drill, and, according to the comparison result, recommends the user whether to integrate or separate the virtual bone guide shape and the virtual implant surgical guide shape.

For example, as illustrated in FIG. 33, if the bone height (e.g., p = 13 mm) at the position of a placed virtual implant does not exceed the maximum length of the guide drill (e.g., 15 mm), the device 1 for designing an implant surgical guide may recommend that the user select between integrated generation of the virtual bone reduction guide shape and the virtual implant surgical guide shape and separate generation of the virtual bone reduction guide shape and the virtual implant surgical guide shape, or may recommend integrated generation. In this case, when the maximum length of the guide drill is longer than all the bone heights at the positions of a plurality of placed virtual implants, the device 1 may recommend that the user select between integrated generation and separate generation of the virtual bone reduction guide shape and the virtual implant surgical guide shape, or may recommend integrated generation.

In contrast, as shown in FIG. 34, when the bone height (e.g., q = 17 mm) at the position of a placed virtual implant exceeds the maximum length of the guide drill (e.g., 15 mm), the device 1 for designing an implant surgical guide may recommend separate generation of the virtual bone reduction guide shape and the virtual implant surgical guide shape, or may proceed to a separate generation step. At this time, when at least any one of the bone heights among the plurality of placed virtual implants exceeds the maximum length of the guide drill, the device 1 may recommend separate generation of the virtual bone reduction guide shape and the virtual implant surgical guide shape, or may proceed to a separate generation step.

Since the separation generation recommendation step has only one option, the device 1 for designing an implant surgical guide may directly proceed to the separate generation step without asking the user whether to make a selection.

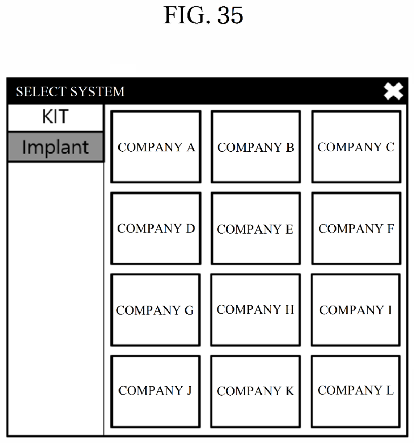

FIG. 35 is a view illustrating a guided surgery kit selection screen according to an embodiment of the present invention.

Referring to FIGS. 1 and 35, the device 1 for designing an implant surgical guide may provide information for a user to select a guided surgery kit or an implant of a predetermined implant company. For example, the information provided may include a diameter of a guide hole for placing a virtual implant, a distance from the uppermost end of the virtual implant to the point where the guide hole is formed, a diameter of the guide drill, and the maximum length of the guide drill. Such information may be stored in a library of the storage unit.

The device 1 designing an implant surgical guide may determine a guided surgery kit by receiving, through a user operation signal, a selection of the guided surgery kit of a predetermined implant company. In another example, the device 1 for designing an implant surgical guide may determine a guided surgery kit of a selected implant company by receiving a selection of the implant company.

Subsequently, the device 1 for designing an implant surgical guide may obtain and check the maximum length information of a guide drill corresponding to the determined guided surgery kit from the storage unit. The maximum length information of the guide drill is used to determine whether to integrate the virtual implant surgical guide shape and the virtual bone reduction guide shape.

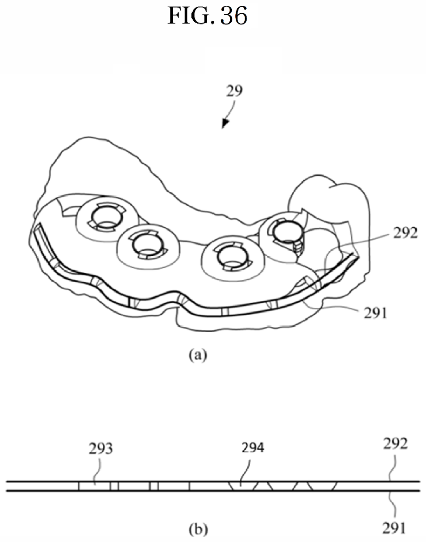

FIG. 36 is a view illustrating a screen for integrating the virtual bone reduction guide shape and the virtual implant surgical guide shape according to an embodiment of the present invention.

More specifically, (a) of FIG. 36 is a view illustrating dental image data 29 in which a bone reduction line and an implant surgical guide line parallel thereto of a virtual bone reduction guide shape and a virtual implant surgical guide shape are represented three-dimensionally, and (b) is a view illustrating a cross-sectional representation of the bone reduction line and the implant surgical guide line parallel thereto.

Referring to FIGS. 1 and 36, the device 1 for designing an implant surgical guide may generate the virtual bone reduction guide shape and the virtual implant surgical guide shape separately, or may generate them in an integrated manner.

First, the process of separately generating the virtual bone reduction guide shape and the virtual implant surgical guide shape will be described below.

In the separate generation step, the device 1 for designing an implant surgical guide generates the virtual implant surgical guide shape including a virtual implant guide hole, and generates the virtual bone reduction guide shape.

Subsequently, the device 1 for designing an implant surgical guide designs the coupled form of the virtual bone reduction guide shape and the virtual implant surgical guide shape. For example, the coupled form of the virtual implant surgical guide shape and the virtual bone reduction guide shape may be designed such that the actual implant surgical guide can be coupled in a manner where the actual implant surgical guide is fitted into the actual bone reduction guide by a physical force of the actual implant surgical guide.

Furthermore, in the case of an edentulous patient, the device 1 for designing an implant surgical guide may generate a virtual anchor pin drilling hole in the virtual bone reduction guide shape in order to fix the bone reduction guide to the bone so that it does not move.

When the virtual bone reduction guide shape and the virtual implant surgical guide shape are generated separately, the two guides must be manufactured individually, resulting in longer manufacturing time and reduced economic efficiency.

Hereinafter, the process of generating the virtual bone reduction guide shape and the virtual implant surgical guide shape in an integrated manner will be described.

The device 1 for designing an implant surgical guide generates the virtual implant surgical guide shape including a virtual implant guide hole, and generates the virtual bone reduction guide shape.

Next, the device 1 for designing an implant surgical guide designs a coupled form of the virtual bone reduction guide shape and the virtual implant surgical guide shape.

Furthermore, in the case of an edentulous patient, the device 1 for designing an implant surgical guide may generate a virtual anchor pin drilling hole in the virtual bone reduction guide shape in order to fix the bone reduction guide to the bone so that it does not move.

The foregoing process is identical to the separate generation process, but the integrated generation process further includes a step of removing a portion of the integrated region so that the portion that guides the bone reduction is clearly exposed.

For example, as shown in FIG. 36, the device 1 for designing an implant surgical guide generates the bone reduction guide line 291 corresponding to a bone reduction line (determined by the bone reduction interface) in the virtual bone reduction guide shape and the virtual implant surgical guide shape. Subsequently, the device 1 for designing an implant surgical guide generates the implant surgical guide line 292 parallel to the bone reduction guide line 291. The bone reduction guide line 291 is parallel to and spaced apart by a predetermined distance from the implant surgical guide line 292. The predetermined distance may be set in advance and may also be changed by the user.

Subsequently, the device 1 for designing an implant surgical guide separates the virtual bone reduction guide shape and the virtual implant surgical guide shape by removing the region between the bone reduction guide line 291 and the implant surgical guide line 292.

Subsequently, the device 1 for designing an implant surgical guide may integrate the virtual bone reduction guide shape and the virtual implant surgical guide shape by connecting the bone reduction guide line 291 and the implant surgical guide line 292 through coupling portions 293 and 294.

In this case, the coupling portion 293 may be a structure of identical shape formed at regular intervals between the bone reduction guide line 291 and the implant surgical guide line 292. In another example, the coupling portion 294 may be formed as an inverted rhombus structure between the bone reduction guide line 291 and the implant surgical guide line 292. The inverted rhombus structure 294 may allow easier placement of a virtual implant and removal of the implant surgical guide shape from the virtual bone reduction guide shape compared to the structure 293 of identical shape formed at regular intervals. However, the structural form is, of course, designed to ensure that stability is not compromised. The process of integrating the virtual bone reduction guide shape and the virtual implant surgical guide shape makes it possible to fabricate two guides at once, thereby shortening the manufacturing time and improving economic efficiency.

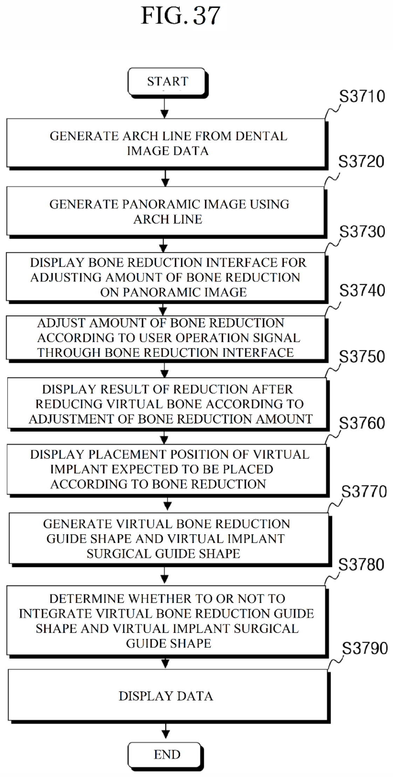

FIG. 37 is a flowchart illustrating a method for designing an implant surgical guide according to an embodiment of the present invention.

Referring to FIGS. 1 and 37, the device 1 for designing an implant surgical guide generates an arch line from dental image data in step S3710.

In the arch line generation step S3710, in the case of partial edentulism, the device 1 for designing an implant surgical guide may segment the corresponding teeth from the dental image data and generate the arch line using the segmented teeth. In the case of edentulism, the device 1 for designing an implant surgical guide may generate the arch line by using a maximum intensity projection image.

Next, in step S3720, the device 1 for designing an implant surgical guide generates a panoramic image by using the generated arch line.

Subsequently, in step S3730, the device 1 for designing an implant surgical guide displays a bone reduction interface for adjusting the amount of bone reduction in the generated panoramic image. The bone reduction interface may be in the form of a line or plane parallel to the occlusal plane in the panoramic image.

Next, in step S3740, the device 1 for designing an implant surgical guide adjusts the amount of bone reduction through the bone reduction interface whose position is adjusted according to a user operation signal. In step S3740, the device 1 for designing an implant surgical guide sets a movable range of the bone reduction interface and adjusts the amount of bone reduction by moving the position of the bone reduction interface (for example, upward and downward) within the set movable range, according to the user operation signal. At this time, the movable range of the bone reduction interface may be within a range that does not invade at least one of anatomical structures among the nerve canal, maxillary sinus, maxilla, and mandible.

Next, in step S3750, the device 1 for designing an implant surgical guide reduces virtual bone according to the adjustment of the bone reduction amount and displays the result of the reduction.

Subsequently, in step S3760, the device 1 for designing an implant surgical guide may display a placement position of a virtual implant expected to be placed according to the bone reduction.

In step S3760 of displaying the virtual implant placement position, the device 1 for designing an implant surgical guide may display a placement position of a virtual implant with respect to the tooth axis of virtual teeth arranged along the arch line or the tooth axis of remaining teeth.

In step S3760 of displaying the virtual implant placement position, the device 1 for designing an implant surgical guide may display the placement position of the virtual implant so as to match a line or plane of the bone reduction interface whose position is adjusted (for example, upward or downward) according to a user operation signal through the bone reduction interface.

In step S3760 of displaying the virtual implant placement position, the device 1 for designing an implant surgical guide may distinguish between non-permissible positions for virtual implant placement and permissible positions for virtual implant placement and display them together. For example, the device 1 for designing an implant surgical guide may display, in a distinguishable manner, non-permissible positions for virtual implant placement and permissible positions for virtual implant placement depending on whether a bone width of a predetermined range is secured when the virtual implant is placed.

In step S3760 of displaying the virtual implant placement position, the device 1 for designing an implant surgical guide may differently display a virtual implant placement position according to the type of virtual implant to be placed. For example, if the virtual implant is a submerged type implant, the device 1 for designing an implant surgical guide may place the virtual implant at a predetermined distance below the line or plane of the bone reduction interface. In contrast, if the virtual implant is a non-submerged type implant, the device 1 for designing an implant surgical guide may place the virtual implant so as to be aligned with the line or plane of the bone reduction interface.

In step S3760 of displaying the virtual implant placement position, the device 1 for designing an implant surgical guide may display a virtual implant placement position on a cross-section perpendicular to the occlusal plane within the range where bone reduction has been performed.

In step S3760 of displaying the virtual implant placement position, the device 1 for designing an implant surgical guide may display the virtual implant placement position in consideration of the diameter of a virtual implant to be placed. For example, the device 1 for designing an implant surgical guide may display an implant diameter adjustment interface for adjusting the diameter of the virtual implant to be placed, and may adjust the implant diameter according to a user operation signal through the displayed implant diameter adjustment interface. The device 1 for designing an implant surgical guide may display a virtual implant placement position according to the adjusted virtual implant diameter.

Furthermore, in step S3760 of displaying the virtual implant placement position, the device 1 for designing an implant surgical guide may display all virtual implant placement positions corresponding to the virtual implant diameters adjusted by virtual implant diameter adjustment. Subsequently, the device 1 for designing an implant surgical guide may select a predetermined virtual implant placement position among the virtual implant placement positions according to the virtual implant diameters in response to a user operation signal.

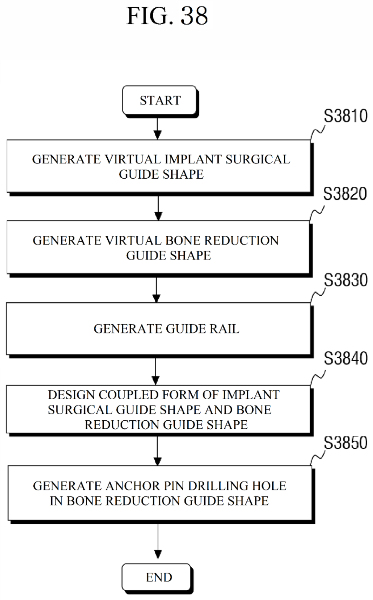

Subsequently, in step S3770, the device 1 for designing an implant surgical guide generates a virtual implant surgical guide shape and a virtual bone reduction guide shape from the dental image data. The process of generating the virtual implant surgical guide shape and the virtual bone reduction guide shape in step S3770 will be described below with reference to FIG. 38.

Subsequently, in step S3780, the device 1 for designing an implant surgical guide may determine and provide whether to integrate the virtual implant surgical guide shape and the virtual bone reduction guide shape.

For example, the device 1 for designing an implant surgical guide obtains length information of a guide drill for implant surgery. Subsequently, the device 1 for designing an implant surgical guide determines whether to integrate the virtual bone reduction guide shape and the virtual implant surgical guide shape according to the length of the guide drill. Subsequently, when separation is determined, the device 1 for designing an implant surgical guide separately generates the virtual bone reduction guide shape and the virtual implant surgical guide shape from the dental image data, for example, from CT data in which the virtual bone has been reduced. Subsequently, the device 1 for designing an implant surgical guide may generate the virtual implant surgical guide shape including a virtual implant guide hole, and may generate the virtual bone reduction guide shape. Subsequently, the device 1 for designing an implant surgical guide may design a coupled form of the virtual bone reduction guide shape and the virtual implant surgical guide shape. Furthermore, a virtual anchor pin drilling hole may be generated in the virtual bone reduction guide shape. Subsequently, the device 1 for designing an implant surgical guide generates the virtual implant surgical guide shape including the virtual implant guide hole and generates the virtual bone reduction guide shape from the dental image data, for example, from second CT data in which the virtual bone has been reduced. Subsequently, the device 1 for designing an implant surgical guide may design a coupled form of the virtual bone reduction guide shape and the virtual implant surgical guide shape. Subsequently, the device 1 for designing an implant surgical guide may generate a virtual anchor pin drilling hole in the virtual bone reduction guide shape.

Subsequently, in step S3790, the device 1 for designing an implant surgical guide displays data including the generated virtual implant surgical guide shape and virtual bone reduction guide shape.

FIG. 38 is a view illustrating a process of generating the virtual implant surgical guide shape and the virtual bone reduction guide shape according to an embodiment of the present invention.

Referring to FIGS. 1, 37, and 38, the device 1 for designing an implant surgical guide, in step S3810, generates the virtual implant surgical guide shape including the virtual implant guide hole.

In step S3810 of generating the virtual implant surgical guide shape, the device 1 for designing an implant surgical guide may generate the virtual implant guide hole at a position spaced apart by a predetermined distance from the uppermost height of the placed virtual implant. In this case, the device 1 for designing an implant surgical guide may generate the virtual implant guide hole such that the axis of the placed virtual implant coincides with the center point of the virtual implant guide hole.

Subsequently, in step S3820, the device 1 for designing an implant surgical guide generates the virtual bone reduction guide shape for guiding bone reduction parallel to the occlusal plane.

In step S3820 of generating the virtual bone reduction guide shape, the device 1 for designing an implant surgical guide may generate an outermost contour line of the virtual bone reduction guide shape on the basis of the position of the virtual implant guide hole.

In step S3820 of generating the virtual bone reduction guide shape, the device 1 for designing an implant surgical guide may generate the outermost contour line of the virtual bone reduction guide shape on the basis of a line or plane of a bone reduction interface whose position is adjusted according to a user operation signal. The bone reduction interface may be in the form of a line or plane parallel to the occlusal plane.

Subsequently, in step S3830, the device 1 for designing an implant surgical guide generates a virtual guide rail having an insertion portion and protruding along the outer periphery of the virtual bone reduction guide shape.

The virtual guide rail may have a form in which a plane of the virtual guide rail is parallel to the occlusal plane and a vertical surface of the virtual guide rail is perpendicular to the occlusal plane. Accordingly, a bone reduction device coupled to an actual guide rail may perform bone reduction in parallel to the occlusal plane.

The guide rail may be generated within a movable range of the bone reduction interface. The movable range of the bone reduction interface may be within a range that does not invade at least one of anatomical structures among the nerve canal, maxillary sinus, maxilla, and mandible.

The guide rail may be formed at a position spaced apart by a predetermined distance vertically and horizontally from the bone reduction interface line.

Subsequently, in step S3840, the device 1 for designing an implant surgical guide designs a coupled form of the virtual implant surgical guide shape and the virtual bone reduction guide shape.

In step S3840 of designing the coupled form, the device 1 for designing an implant surgical guide may design the coupled form of the virtual implant surgical guide shape and the virtual bone reduction guide shape such that, when the actual implant surgical guide is fastened to the actual bone reduction guide, the actual implant surgical guide is coupled in a manner where the actual implant surgical guide is fitted into the actual bone reduction guide by physical force.

In step S3840 of designing the coupled form, the device 1 for designing an implant surgical guide may design the coupled form of the virtual implant surgical guide shape and the virtual bone reduction guide shape such that the actual bone reduction guide is mounted in a cylindrical groove of the actual implant surgical guide.

In step S3840 of designing the coupled form, the device 1 for designing an implant surgical guide may design the coupled form of the virtual implant surgical guide shape and the virtual bone reduction guide shape such that the actual implant surgical guide and the actual bone reduction guide are coupled in an omega shape.

Furthermore, in step S3850, the device 1 for designing an implant surgical guide may generate the virtual anchor pin drilling hole in the virtual bone reduction guide shape.

So far, the present invention has been described with reference to embodiments thereof. It will be understood by those skilled in the art that various changes in form and details may be made therein without departing from the essential features of the present invention. Therefore, the disclosed embodiments should be considered in descriptive sense only and not for purposes of limitation. The scope of the present invention should be defined not by the detailed description but by the appended claims, and all differences falling within a scope equivalent to the claims should be construed as being encompassed by the present invention.

## Claims

1. A method for designing an implant surgical guide using a device for designing the implant surgical guide, the method comprising the steps of:
placing a virtual implant into dental image data;
generating a virtual implant surgical guide shape including a virtual implant guide hole;
generating a virtual bone reduction guide shape for guiding bone reduction parallel to an occlusal plane;
generating a virtual guide rail having an insertion portion and protruding along an outer periphery of the virtual bone reduction guide shape; and
designing a coupled form of the virtual implant surgical guide shape and the virtual bone reduction guide shape.

2. The method of claim 1, wherein the step of placing the virtual implant comprises displaying a placement position of the virtual implant so as to match a line or plane of a bone reduction interface whose position is adjusted in accordance with a user operation signal through the bone reduction interface.

3. The method of claim 2, wherein the bone reduction interface is in a form of a line or plane parallel to the occlusal plane.

4. The method of claim 1, wherein the step of generating the virtual implant surgical guide shape comprises forming the virtual implant guide hole at a position spaced apart by a predetermined distance from a top end portion of the placed virtual implant.

5. The method of claim 4, wherein the step of generating the virtual implant surgical guide shape comprises forming the virtual implant guide hole such that an axis of the placed virtual implant coincides with a center point of the virtual implant guide hole.

6. The method of claim 1, wherein the step of generating the virtual bone reduction guide shape comprises generating an outermost contour line of the virtual bone reduction guide shape on a basis of a position of the virtual implant guide hole.

7. The method of claim 1, wherein the step of generating the virtual bone reduction guide shape comprises generating an outermost contour line of the virtual bone reduction guide shape on a basis of a line or plane of the bone reduction interface whose position is adjusted in response to a user operation signal.

8. The method of claim 1, wherein the virtual guide rail has a plane parallel to the occlusal plane and a vertical surface perpendicular to the occlusal plane.

9. The method of claim 1, wherein the virtual guide rail is generated within a movable range of a bone reduction interface and the movable range of the bone reduction interface is within a range that does not invade at least one of anatomical structures among a nerve canal, maxillary sinus, a maxilla, and a mandible.

10. The method of claim 1, wherein the virtual guide rail is formed at a position spaced apart by a predetermined distance vertically and horizontally from a bone reduction interface line or plane.

11. The method of claim 1, wherein the step of designing the coupled form comprises designing the coupled form of the virtual implant surgical guide shape and the virtual bone reduction guide shape such that, when an actual implant surgical guide is fastened to an actual bone reduction guide, the actual implant surgical guide is coupled in a manner where the actual implant surgical guide is fitted into the actual bone reduction guide by physical force.

12. The method of claim 1, wherein the step of designing the coupled form comprises designing the coupled form of the virtual implant surgical guide shape and the virtual bone reduction guide shape such that an actual bone reduction guide is mounted into a cylindrical groove of an actual implant surgical guide.

13. The method of claim 1, wherein the step of designing the coupled form comprises designing the coupled form of the virtual implant surgical guide shape and the virtual bone reduction guide shape such that an actual bone reduction guide and an actual implant surgical guide are coupled in an omega shape.

14. The method of claim 1, further comprising the step of generating a virtual anchor pin drilling hole in the virtual bone reduction guide shape.

15. The method of claim 1, further comprising the steps of:
inserting a guiding mechanism of a bone reduction device into an actual insertion portion of an actual guide rail manufactured by outputting the virtual guide rail;
moving the guiding mechanism of the bone reduction device, inserted into the insertion portion, along a movement path constrained by the actual guide rail; and
reducing bone protruding from an opening of the actual bone reduction guide, with a cutting mechanism coupled to the guiding mechanism moving together along the movement path.

16. The method of claim 1, further comprising the steps of:
obtaining length information of a guide drill for implant surgery;
determining whether to integrate the virtual bone reduction guide shape and the virtual implant surgical guide according to the obtained length of the guide drill;
in response to determining to integrate the virtual bone reduction guide shape and the virtual implant surgical guide shape, generating the virtual bone reduction guide shape and the virtual implant surgical guide shape in an integrated manner from the dental image data; and
displaying the generated data.

17. The method of claim 16, wherein the step of determining whether to integrate comprises the steps of:
determining a bone height before bone reduction with respect to a position of the virtual implant placed in the dental image data;
checking the length of the guide drill; and
comparing the bone height and the length of the guide drill and, according to a comparison result, recommending whether to integrate or separate the virtual bone reduction guide shape and the virtual implant surgical guide.

18. The method of claim 16, wherein the step of generating in an integrated manner comprises the steps of:
generating the virtual implant surgical guide shape including the virtual implant guide hole;
generating the virtual bone reduction guide shape;
designing a coupled form of the virtual bone reduction guide shape and the virtual implant surgical guide shape;
generating a bone reduction guide line corresponding to a bone reduction line in the virtual bone reduction guide shape and the virtual implant surgical guide shape;
generating an implant surgical guide line parallel to and at a predetermined distance away from the bone reduction guide line;
separating the virtual bone reduction guide shape and the virtual implant surgical guide shape by removing a region between the bone reduction guide line and the implant surgical guide line; and
integrating the virtual bone reduction guide shape and the virtual implant surgical guide shape by combining the bone reduction guide line and the implant surgical guide line through a coupling portion.

19. A device for designing an implant surgical guide, comprising:
a data acquisition unit configured to obtain dental image data for implant surgery;
a control unit configured to generate a virtual implant surgical guide shape including a virtual implant guide hole, to generate a virtual bone reduction guide shape for guiding bone reduction parallel to the occlusal plane, to generate a virtual guide rail protruding with an insertion portion along an outer periphery of the virtual bone reduction guide shape, and to design a coupled form of the virtual implant surgical guide shape and the virtual bone reduction guide shape; and
a display unit configured to display data including the virtual implant surgical guide shape and the virtual bone reduction guide shape.
